(19)

Europäisches Patentamt

European Patent Office

Office européen des brevets

(11) **EP 3 020 417 A1**

(12) **EUROPEAN PATENT APPLICATION**
published in accordance with Art. 153(4) EPC

(43) Date of publication:
**18.05.2016 Bulletin 2016/20**

(21) Application number: **14822934.7**

(22) Date of filing: **10.07.2014**

(51) Int Cl.:
*A61K 47/48* (2006.01)  *A61K 31/573* (2006.01)
*A61K 47/36* (2006.01)  *A61K 47/38* (2006.01)
*A61P 11/00* (2006.01)  *A61P 11/08* (2006.01)
*A61P 25/02* (2006.01)  *A61P 37/08* (2006.01)
*A61P 43/00* (2006.01)

(86) International application number:
**PCT/JP2014/068513**

(87) International publication number:
**WO 2015/005459 (15.01.2015 Gazette 2015/02)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **10.07.2013  JP 2013144365**

(71) Applicant: **Seikagaku Corporation
Tokyo 100-0005 (JP)**

(72) Inventors:
• **SATO, Tomoya
Tokyo 100-0005 (JP)**
• **TAKEUCHI, Hisayuki
Tokyo 100-0005 (JP)**
• **ZUINEN, Ryoji
Tokyo 100-0005 (JP)**

(74) Representative: **Hoffmann Eitle
Patent- und Rechtsanwälte PartmbB
Arabellastraße 30
81925 München (DE)**

(54) **PHARMACEUTICAL COMPOSITION FOR RESPIRATORY ADMINISTRATION**

(57)     The present invention provides a pharmaceutical composition for respiratory administration containing a polysaccharide derivative having a group derived from a polysaccharide and a group derived from a physiologically active substance that is covalently bonded to the group derived from a polysaccharide.

EP 3 020 417 A1

**Description**

TECHNICAL FIELD

The present invention relates to a pharmaceutical composition for respiratory administration.

BACKGROUND ART

As a pharmaceutical preparation administered to a respiratory system, a preparation for intrapulmonary administration and a preparation for intranasal administration are mainly known.

Since the inner surface area of pulmonary alveoli is almost equal to the surface area of small intestine, the preparation for intrapulmonary administration has good absorption efficiency and, as the absorbed pharmaceuticals directly enter a systemic circulatory system from heart, there is an advantage that it can avoid the liver first-pass effect. Furthermore, as the working effects are rapidly exhibited due to fast absorption, it has been conventionally used as a method for administering a bronchodilator during an asthma attack.

In addition to the above, a medication management therapy including prophylactic inhalation administration of low-dose steroid for an extended period of time is recently employed, and it exhibits clinically remarkable results.

Furthermore, for the purpose of treating chronic obstructive pulmonary disorder (hereinafter, also referred to as COPD), a bronchodilator preparation for intrapulmonary administration is also known.

Meanwhile, as it is necessary to prevent short breath during inhalation, it is difficult for a preparation for intrapulmonary administration itself to have a large pharmaceutical amount. In addition, as the pharmaceutical is adhered onto a mouth cavity, a pharynx, or the like, only a small amount of the pharmaceutical can actually reach the target site. In addition, as the pharmaceutical is diffused absorbed in an alveolus based on passive transport, a pharmaceutical with high oil-solubility is known to be absorbed better while a fine · minor pharmaceutical is easily lost from an administration site.

The preparation for intranasal administration can be surely administered in a relatively large amount to a target site and, as the vascular system present under nasal mucous membrane has a large blood flow, the pharmaceutical can be easily delivered to the vein. Thus, like the intrapulmonary absorption, it is advantageous in that it can avoid the liver first-pass effect. Furthermore, for the purpose of treating seasonal or perennial allergic rhinitis, various anti-inflammatory agents are recently used as a preparation for intranasal administration, and in particular, a steroid agent having reduced side effects in a human body is widely used. However, like the preparation for intrapulmonary administration, the preparation for intranasal administration is also lost easily from an administration site after the administration due to ciliary beating.

As described above, although a pharmaceutical preparation for administration to a respiratory system is quite widely used in these days, any of those preparations needs to be administered 1 to 4 times or so per day.

For a disorder like asthma, COPD, and perennial allergic rhinitis, a pharmaceutical needs to be administered over a lifetime until symptom remission is observed. Accordingly, a pharmaceutical preparation with higher convenience for allowing reduced administration frequency based on long-acting effect needs to be developed. However, development of a pharmaceutical preparation fully satisfying such needs has not been sufficiently made, and currently, it is required to have the administration every day.

In relation to the above, in JP 2008-156327 A, application of a mixture of fluticasone and chondroitin sulfate to a pulmonary disease is disclosed.

Meanwhile, in "Preparation of Chondroitin sulfate-Prednisolone Conjugate and In Vitro Characteristics", P24-12 of 27th Lecture Synopsis of The Academy of Pharmaceutical Science and Technology, Japan (by Matsuyama Mototaka, Shumoku Tomoyuki, and Onishi Hiraku, May 24, 2012), use of a polysaccharide-coupled pharmaceutical for treating gonarthrosis is disclosed.

CITATION LIST

Patent Literatures

Patent Literature 1: JP 2008-156327 A

Non Patent Literature

Non Patent Literature 1: "Preparation of Chondroitin sulfate-Prednisolone Conjugate and In Vitro Characteristics", P24-12 of 27th Lecture Synopsis of The Academy of Pharmaceutical Science and Technology, Japan (by Matsuyama Mototaka, Shumoku Tomoyuki, and Onishi Hiraku, May 24,2012).

SUMMARY OF INVENTION

Problem to be Solved by the Invention

The inhalation pharmaceutical product used for treatment of a respiratory disorder needs to be inhaled everyday, 1 to 4 times per day. For asthma, the pharmaceutical needs to be taken until the symptom remission is observed. For COPD, it needs to be taken over a lifetime. For allergic rhinitis, it also needs to be administered everyday for an extended period of time, if it is perennial rhinitis. Thus, it is a heavy burden on a patient due to highly cumbersome process. For such reasons, there are many cases in which a patient himself stops taking the medicines as soon as the symptom is slightly alleviated, yielding a huge problem of low adherence. The low adherence significantly reduces the control of symptoms and it becomes a cause of having symptoms in severe and incurable state. Thus, there has been a demand for development of a pharmaceutical preparation enabling improved adherence.

Furthermore, compared to general steroids, although it is less likely for the steroids used for treatment of a respiratory disorder to exhibit side effects, when they are used continuously for an extended period of time, not only local side effects like oral candidiasis and husky voice but also systemic side effects like a problem in eye (cataract and glaucoma), a problem in skin (skin thinning, easy bleeding), suppressed function of hypothalamus · pituitary · adrenal gland, and problem in bone (osteoporosis) may occur.

The present invention is devised in view of the above problems to be solved, and an object of the invention is to provide a pharmaceutical composition for respiratory administration which can continuously release a physiologically active substance over an extended period of time.

Means for Solving the Problem

Considering the above problems to be solved, the present inventors conducted intensive studies to develop a pharmaceutical preparation for inhalation with extended working time, and as a result, it was found that, by using a polysaccharide derivative having a physiologically active substance introduced to polysaccharides via a covalent bond, the working time of the physiologically active substance can be extended compared to a pharmaceutical composition of a related art and the amount of the physiologically active substance itself can be greatly reduced. The present invention is completed accordingly. Namely, specific means for solving the problems described above are as follows and the following embodiments are encompassed by the present invention.

<1> A pharmaceutical composition for respiratory administration containing a polysaccharide derivative having a group derived from a polysaccharide and a group derived from a physiologically active substance that is covalently bonded to the group derived from a polysaccharide.

<2> The pharmaceutical composition described in <1>, in which the group derived from a polysaccharide and the group derived from a physiologically active substance are covalently bonded with a spacer therebetween.

<3> The pharmaceutical composition described in <2>, in which the group derived from a physiologically active substance and the spacer are covalently bonded through an ester bond.

<4> The pharmaceutical composition described in <2> or <3>, in which the group derived from a polysaccharide and the spacer are covalently bonded through an amide bond.

<5> The pharmaceutical composition described in any one of <2> to <4>, in which the spacer is a divalent or higher valent group that is derived from at least one selected from a group consisting of an amino acid, amino alcohol, dicarboxylic acid, and a derivative thereof.

<6> The pharmaceutical composition described in <5>, in which the spacer is a divalent group derived from ω-aminofatty acid which may have a substituent group.

<7> The pharmaceutical composition described in any one of <1> to <6>, in which the polysaccharide is at least one selected from a group consisting of glycosaminoglycan and homoglycan.

<8> The pharmaceutical composition described in any one of <1> to <7>, in which the polysaccharide is at least one selected from a group consisting of hyaluronic acid, chondroitin sulfate, chitosan, and carboxymethyl cellulose.

<9> The pharmaceutical composition described in any one of <1> to <8>, in which dissociation rate of the physiologically active substance from the polysaccharide derivative is 0.1 to 30%/day in phosphate buffered saline with pH of 7.5 at 36°C.

<10> The pharmaceutical composition described in any one of <1> to <9>, in which the physiologically active substance is at least one selected from a group consisting of a steroid, a bronchodilator, an anti-allergic drug, and an anti-choline drug.

<11> The pharmaceutical composition described in any one of <1> to <10>, which is used for treatment of a respiratory disease.

Advantageous Effects of the Invention

According to the present invention, a pharmaceutical composition for respiratory administration which can continuously release a physiologically active substance over an extended period of time can be provided.

BRIEF DESCRIPTION OF THE DRAWINGS

Fig. 1 is a graph illustrating the correlation between the hydropathy index of amino acids and dissociation rate.

Fig. 2 is a graph illustrating the time-lapse change in concentration of betamethasone that is dissociated in pulmonary tissue.

Fig. 3 is a graph illustrating the time-lapse change in concentration of betamethasone that is dissociated in plasma.

Fig. 4 is a graph illustrating the number of eosinophils in alveolar lavage fluid.

Fig. 5 is a graph illustrating the correlation between the dissociation rate and dissociation concentration of a pharmaceutical.

Fig. 6 is a graph illustrating the dose responsiveness of budesonide alone in an OVA sensitized rat model.

Fig. 7 is a graph illustrating one example of the dose responsiveness of polysaccharide derivatives in an OVA sensitized rat model.

Fig. 8 is a graph illustrating continuous inhibitory effect of polysaccharide derivatives on augmented airway hyper responsiveness.

Fig. 9 is a graph illustrating average cough number in a rhinitis model from the administration day to Day 7 after administering a pharmaceutical preparation.

Fig. 10 is a graph illustrating total of average cough number in a rhinitis model during eight days, i.e., from the administration day to Day 7 after administering a pharmaceutical preparation.

MODE FOR CARRYING OUT THE INVENTION

As described herein, the numerical range described by using "to" indicates a range which includes the numbers described before and after the "to" as a minimum value and a maximum value, respectively. In addition, when plural materials corresponding to each component are present in the composition, content of each component in the composition means the total amount of those plural materials in the composition, unless it is specifically described otherwise.

Hereinbelow, the present invention is explained in view of the embodiments of invention.

<Pharmaceutical composition>

The pharmaceutical composition of this embodiment is a pharmaceutical composition for respiratory administration containing at least one polysaccharide derivative having a group derived from a polysaccharide and a group derived from a physiologically active substance that is covalently bonded to the group derived from a polysaccharide (hereinbelow, also referred to as a pharmaceutical). If necessary, the pharmaceutical composition may contain an additive like pharmaceutically acceptable vehicle.

By containing a polysaccharide derivative with a structure in which a polysaccharide and a physiologically active substance are linked to each other by a covalent bond that can be decomposed in a living body, the pharmaceutical composition can continuously release the physiologically active substance over an extended period of time when administered to a respiratory system. Furthermore, based on reduced administration frequency and improvement of inconvenient administration, adherence can be improved. Furthermore, side effects can be reduced based on reduced number of application frequency. Still furthermore, the amount of the required physiologically active substance itself can be greatly reduced.

(Polysaccharide derivative)

The polysaccharide derivative has a group derived from a polysaccharide and a group derived from a physiologically active substance that is covalently bonded to the group derived from a polysaccharide. With regard to the polysaccharide derivative, as the covalent bond between a group derived from a polysaccharide and a group derived from a physiologically active substance is dissociated (preferably, solvolyzed), the physiologically active substance is dissociated. The polysaccharide derivative may further have other substituent groups, if necessary. Furthermore, when the polysaccharide derivative has an acidic group like a carboxy group and a sulfuric acid group, it may be in a free acid state without forming a salt or in a state of a pharmaceutically acceptable salt.

As the pharmaceutically acceptable salt, there may be mentioned, for example, a salt with an alkali metal ion such as a sodium salt or a potassium salt, a salt with an alkaline earth metal ion such as a magnesium salt or a calcium salt. The polysaccharide derivative is, from the viewpoint of applicability to a living body and affinity therefor, preferably a salt with a pharmaceutically acceptable alkali metal ion, and more preferably a sodium salt.

With regard to the polysaccharide derivative, the covalent bond between a group derived from a polysaccharide and a group derived from a physiologically active substance include a first embodiment in which a group derived from a polysaccharide and a group derived from a physiologically active substance are directly bonded to each other via a covalent bond and a second embodiment in which a group derived from a polysaccharide and a group derived from a physiologically active substance are covalently bonded to each other with a spacer therebetween. Meanwhile, forming a covalent bond with a spacer therebetween means a state in which, as the spacer is a divalent or higher valent group and each of at least one group derived from a polysaccharide and at least one group derived from a physiologically active substance is covalently bonded to a spacer, the group derived from a polysaccharide and the group derived from a physiologically active substance are linked to each other with a spacer therebetween. The spacer is not particularly limited either, if it is a divalent or higher valent group. It is preferably a divalent group.

The covalent bond between a group derived from a polysaccharide and a group derived from a physiologically active substance of the first embodiment is, from the viewpoint of controlling the dissociation rate of a physiologically active substance from a polysaccharide derivative, preferably a covalent bond which can be decomposed in a living body and formed by condensation reaction, and it is more preferably an ester bond. According to the first embodiment, as the covalent bond between a group derived from a polysaccharide and a group derived from a physiologically active substance is decomposed (preferably, solvolyzed), the physiologically active substance is dissociated.

Furthermore, the covalent bond between a group derived from a physiologically active substance and a spacer of the second embodiment is, from the viewpoint of controlling the dissociation rate of a physiologically active substance from a polysaccharide derivative, preferably a covalent bond which can be decomposed in a living body and formed by condensation reaction, and it is more preferably an ester bond. According to the second embodiment, as the covalent bond between a group derived from a physiologically active substance and a spacer is decomposed (preferably, solvolyzed), the physiologically active substance is dissociated. Meanwhile, according to the second embodiment, it is also possible that, after decomposition of a covalent bond between a group derived from a polysaccharide and a spacer, the covalent bond between a group derived from a physiologically active substance and a spacer is also decomposed to dissociate a physiologically active substance.

Namely, the polysaccharide derivative is preferably a compound in which a group derived from a polysaccharide and a group derived from a physiologically active substance are linked to each other via a covalent bond including an ester bond.

As described herein, the group derived from a polysaccharide means a group formed by removing at least one selected from a group consisting of a hydroxyl group and a hydrogen atom from a polysaccharide molecule. Furthermore, the group derived from a physiologically active substance is a group formed by removing at least one selected from a group consisting of a hydroxyl group and a hydrogen atom from a physiologically active substance. Meanwhile, the site in a polysaccharide molecule or a physiologically active substance from which a hydroxyl group or a hydrogen atom is removed is not particularly limited. For example, when a hydroxyl group is removed, it is preferable that a hydroxyl group is removed from a carboxy group or a sulfuric acid group which may be found in a polysaccharide molecule or a physiologically active substance. More preferably, a hydroxyl group is removed from a carboxy group. Furthermore, when a hydrogen atom is removed, for example, it is preferable that a hydrogen atom is removed from a hydroxyl group or an amino group which may be found in a polysaccharide molecule or a physiologically active substance.

The polysaccharide molecule constituting a polysaccharide derivative is not particularly limited as long as it is a compound in which plural monosaccharide molecules are linked through a glycoside bond. Number of the monosaccharide molecule constituting a polysaccharide molecule is 2 or more, and more preferably 10 or more.

Examples of the monosaccharide molecule constituting a polysaccharide molecule include D-glucose, β-D-glucuronic acid, α-L-iduronic acid, D-glucosamine, α-D-galactosamine, and a derivative thereof. Examples of the monosaccharide derivative include an acetylation product, a sulfate ester product, a methyl carboxylation product, and an alkylation product.

Among them, the monosaccharide is preferably at least one selected from a group consisting of D-glucose, β-D-glucuronic acid, α-L-iduronic acid, D-glucosamine, and a derivative thereof.

The polysaccharide molecule can be also a compound in which a substituent group is further added to a compound having monosaccharide molecules linked through a glycoside bond. Examples of the substituent group include an alkyl group with 1 to 4 carbon atoms, a carboxymethyl group, an acetyl group, and a sulfate group. The substituent group may be used either singly or in combination of two or more types.

The polysaccharide molecule may be a homoglycan consisting of one type of a monosaccharide molecule or a heteroglycan consisting of two or more types of a monosaccharide molecule. When the polysaccharide molecule is a homoglycan, it is preferably a homoglycan derivative which has a substituent group such as a carboxymethyl group or an acetyl group. Examples of the monosaccharide molecule constituting a homoglycan include D-glucose and D-glucosamine.

When the polysaccharide molecule is a heteroglycan, it is preferably glycosaminoglycan having an aminosugar (galactosamine, glucosamine, or the like), and uronic acid (glucuronic acid, iduronic acid, or the like) or galactose as a constitutional unit (hereinbelow, also referred to as a "GAG"). Examples of the glycosaminoglycan include hyaluronic acid (hereinbelow, also referred to as "HA"), chondroitin sulfate (hereinbelow, also referred to as "CS"), heparin, heparan sulfate, keratan sulfate, and dermatan sulfate.

From the viewpoint of controlling the dissociation rate of a physiologically active substance from a polysaccharide derivative, the polysaccharide molecule is preferably at least one selected from a group consisting of glycosaminoglycan and homoglycan which may have a substituent group. Among them, from the viewpoint of controlling the dissociation rate of a physiologically active substance from a polysaccharide derivative, it is more preferably a polysaccharide molecule having, in a constitutional unit, at least one reactive functional group selected from a group consisting of a carboxy group and an amino group.

Examples of the polysaccharide molecule include hyaluronic acid, chondroitin sulfate, heparin, heparan sulfate, keratan sulfate, dermatan sulfate, carboxymethyl cellulose (hereinbelow, also referred to as "CMC"), carboxymethylethyl cellulose, chitosan, chitin, cellulose, and a derivative thereof.

Among them, it is more preferably at least one selected from a group consisting of hyaluronic acid, chondroitin sulfate, chitosan, and carboxymethyl cellulose.

Depending on a type of the molecule, the polysaccharide molecule can be produced by a known method. Molecular weight of the polysaccharide molecule is not particularly limited, and it is suitably selected depending on the type of a monosaccharide molecule for constituting the polysaccharide, purpose, or the like.
The weight average molecular weight of a polysaccharide molecule can be 10,000 to 5,000,000 or 10 kDa to 5,000 kDa, for example. It is preferably 15,000 to 2,000,000 or 15 kDa to 2,000 kDa, and more preferably 20,000 to 1,000,000 or 20 kDa to 1,000 kDa. The weight average molecular weight of a polysaccharide molecule can be measured by a common method using GPC.

Furthermore, the desired polysaccharide can be also selected depending on solution viscosity as a substituent index for molecular weight.

When hyaluronic acid is used as a polysaccharide molecule, the weight average molecular weight of hyaluronic acid is not particularly limited, and it can be suitably selected depending on the purpose (e.g., disorder for application). The weight average molecular weight can be 10,000 to 5,000,000 or 10 kDa to 5,000 kDa, for example. From the viewpoint of production efficiency, it is preferably 50,000 to 3,000,000 or 50 kDa to 3,000 kDa, and more preferably 200,000 to 1,000,000 or 200 kDa to 1,000 kDa.

When chondroitin sulfate is used as a polysaccharide molecule, type of the chondroitin sulfate is not particularly limited, and any chondroitin sulfate like chondroitin sulfate A, chondroitin sulfate B, chondroitin sulfate C, chondroitin sulfate D, and chondroitin sulfate E can be used. Type of the chondroitin sulfate can be suitably selected depending on the purpose (e.g., disorder for application). The weight average molecular weight of the chondroitin sulfate is not particularly limited, and it can be selected depending on the purpose or the like. For example, the weight average molecular weight is preferably 10,000 to 100,000 or 10 kDa to 100 kDa, more preferably 10,000 to 40,000 or 10 kDa to 40 kDa, still more preferably 15,000 to 30,000 or 15 kDa to 30 kDa, and particularly preferably 20,000 to 30,000 or 20 kDa to 30 kDa.

When carboxymethyl cellulose, carboxymethyl ethyl cellulose, or the like are used as a polysaccharide molecule, the viscosity (molecular weight) and etherification degree (substitution degree) are not particularly limited, and they can be suitably selected depending on the purpose (e.g., disorder for application). It is preferable that the etherification degree is 0.6 to 1.3 and viscosity is 100 to 18,000 (mPa·s) for 1% by mass aqueous solution. It is more preferable that the etherification degree is 0.8 to 1.0 and viscosity is 1,000 to 6,000 (mPa·s) for 1% by mass aqueous solution. The viscosity can be measured by using B type rotational viscometer at 25°C condition.

When chitosan is used as a polysaccharide molecule, the viscosity (molecular weight) and de-acetylation degree (substitution degree) are not particularly limited, and they can be suitably selected depending on the purpose (e.g., disorder for application). It is preferable that the deacetylation degree is 70% or more and viscosity is 5 to 1,000 (mPa·s) for 1 % by mass acetic acid solution. It is more preferable that the deacetylation degree is 80% or more and viscosity is 10 to 500 (mPa·s) for 0.5% by mass acetic acid solution. The viscosity can be measured by using B type rotational viscometer with 0.5% by mass acetic acid solution at 20°C condition.

The polysaccharide derivative has at least one group derived from a physiologically active substance. Type of the physiologically active substance for constituting a polysaccharide derivative is not particularly limited if it is a physiologically active substance that can be applied for respiratory administration, and it can be suitably selected depending on a purpose or the like. The physiological activity possessed by a physiologically active substance can be a physiological activity for treatment, prevention, or the like of a disorder, or a physiological activity for diagnosis or the like. Specific examples of the physiological activity can include a steroidal activity, a $\beta_2$ adrenaline receptor stimulation activity, a theophylline type activity, an anti-viral activity, an anti-allergic activity, an anti-choline activity, an anti-inflammatory activity, an anti-bacterial activity, an expectorant activity, an anti-oxidant activity, and an interferon type activity.

Furthermore, the physiologically active substance is preferably a compound which has at least one of a carboxy group and a hydroxyl group, from the viewpoint of controlling the dissociation rate from a polysaccharide derivative. More preferably, it is a compound having at least a hydroxyl group.

Specific examples of the physiologically active substance can include steroids, a bronchodilator, an anti-viral drug, an anti-allergic drug, an anti-choline drug, an anti-bacterial drug, an anti-inflammatory drug, an expectorant, an anti-oxidant, and an interferon agent. It is preferably at least one selected from a group consisting of steroids, a bronchodilator, an anti-allergic drug, and an anti-choline drug.

Type of the steroids is not particularly limited, and it can be suitably selected depending on purpose or the like. Specific examples thereof include betamethasone, prednisolone, budesonide, and fluticasone. It is preferable that those steroids are suitably selected in consideration of disorders for application, activity strength of steroid itself, influence of side effects, or the like.

Type of the bronchodilator is not particularly limited, and it can be suitably selected depending on purpose or the like. Specific examples of the bronchodilator include a $\beta_2$ adrenaline receptor stimulator, a theophylline drug (i.e., xanthine derivatives), and a parasympatholytic agent.

Specific examples of the $\beta_2$ adrenaline receptor stimulator include tulobuterol. Furthermore, specific examples of the xanthine derivatives include proxyphylline. Examples of the parasympatholytic agent include an anti-cholinergic drug that is described below.

Type of the anti-allergic drug is not particularly limited, and it can be suitably selected depending on purpose or the like. Specific examples of the anti-allergic drug include a drug for suppressing dissociation of chemical delivery substance, a histamine antagonist, a thromboxane synthesis inhibitor, a thromboxane antagonist, a $TH_2$ cytokine inhibitor, and a leukotriene antagonist.

Specific examples of the thromboxane antagonist include seratrodast. Specific examples of the leukotriene antagonist include montelukast.

Type of the anti-cholinergic drug is not particularly limited, and it can be suitably selected depending on purpose or the like. Specific examples of the anti-cholinergic drug include ipratropium.

In the polysaccharide derivative, a group derived from a polysaccharide and a group derived from a physiologically active substance may be directly bonded to each other via a covalent bond, or a group derived from a polysaccharide and a group derived from a physiologically active substance are covalently bonded to each other with a spacer therebetween. However, from the viewpoint of easier control of the dissociation rate of a physiologically active substance from a polysaccharide derivative, it is preferable that a group derived from a polysaccharide and a group derived from a physiologically active substance are covalently bonded to each other with a spacer therebetween.

In the polysaccharide derivative, when a group derived from a polysaccharide and a group derived from a physiologically active substance are directly bonded to each other via a covalent bond (first embodiment), from the viewpoint of control of the dissociation rate of a physiologically active substance from a polysaccharide derivative, it is preferable that the polysaccharide derivative is a compound in which a polysaccharide and a physiologically active substance are bonded by condensation. More preferably, it is a compound in which a polysaccharide and a physiologically active substance are bonded through an ester bond. Even more preferably, it is a compound in which an ester bond is formed between a carboxy group of a polysaccharide and a hydroxyl group of a physiologically active substance.

In a case of the first embodiment, by controlling the dissociation property (preferably, hydrolysis property) of a covalent bond between a group derived from a polysaccharide and a group derived from a physiologically active substance, the dissociation rate of a physiologically active substance from a polysaccharide derivative can be controlled. Specifically, for example, it is possible that a compound having an electron withdrawing group near the hydroxyl group like hydroxymethyl carbonyl group is selected as a physiologically active substance and glycosaminoglycan or homoglycan having a carboxy group (preferably, glycosaminoglycan) is selected as a polysaccharide. Accordingly, dissociation rate of a desired physiologically active substance can be easily obtained.

Controlling the dissociation property of the covalent bond between a group derived from a polysaccharide and a group derived from a physiologically active substance can be also controlled by selecting the position at which the group derived from a polysaccharide binds to the physiologically active substance. For example, by having the group derived from a polysaccharide bonded to a sterically hindered site in the physiologically active substance, the dissociation rate of a physiologically active substance from a polysaccharide derivative can be lowered. Specifically, for example, when a steroid is used as a physiologically active substance, the hydroxyl group at position 11 can be a binding site so that the dissociation rate is lowered compared to a case in which the hydroxyl group at position 21 is a binding site.

In the polysaccharide derivative, when a group derived from a polysaccharide and a group derived from a physiologically active substance are covalently bonded to each with a spacer therebetween (second embodiment), from the viewpoint of controlling the dissociation rate of a physiologically active substance from a polysaccharide derivative, it is preferable that at least one of a group derived from a polysaccharide and a spacer and a group derived from a physiologically active substance and a spacer in the polysaccharide derivative are covalently bonded through an ester bond. It is more preferable that a group derived from a physiologically active substance and a spacer are covalently bonded through an ester bond. It is even more preferable that a group derived from a polysaccharide and a spacer are covalently bonded through an amide bond and a group derived from a physiologically active substance and a spacer are covalently bonded through an ester bond.

Furthermore, in the polysaccharide derivative of the second embodiment, it is also possible that two or more groups derived from a physiologically active substance are covalently bonded to a group derived from a polysaccharide molecule with one spacer therebetween. The number of the group derived from a physiologically active substance which covalently binds to one spacer is not particularly limited, if it is 1 or higher.

Namely, the compound for forming a spacer (hereinbelow, it is also referred to as a spacer-forming molecule) preferably has, in the molecular structure, two or more reactive functional groups that are selected from a group consisting of a carboxy group, a hydroxyl group and an amino group and a coupling group which couples those two or more reactive functional groups.

Herein, the two or more reactive functional groups that are selected from a group consisting of a carboxy group, a hydroxyl group and an amino group are suitably selected in accordance with a structure of a polysaccharide molecule and a physiologically active substance. For example, when a carboxy group is contained in a polysaccharide molecule, the spacer-forming molecule preferably has a hydroxyl group or an amino group as a response. More preferably, it has an amino group. When an amino group or a hydroxyl group is contained in a polysaccharide molecule, the spacer-forming molecule preferably has a carboxy group as a response.

Meanwhile, when a carboxy group is contained in a physiologically active substance, the spacer-forming molecule preferably has a hydroxy group or an amino group as a response. More preferably, it has a hydroxy group. When a hydroxy group is contained in a physiologically active substance, the spacer-forming molecule preferably has a carboxy group as a response.

From the viewpoint of controlling dissociation rate of a physiologically active substance from a polysaccharide derivative, the spacer-forming molecule is preferably at least one selected from a group consisting of a compound having a carboxy group, an amino group, and a coupling group (preferably, an amino acid), a compound having a hydroxy group, an amino group, and a coupling group (preferably, an amino alcohol), a compound having two or more carboxy groups and a coupling group (preferably, dicarboxylic acid), a compound having a carboxy group, a hydroxy group, and a coupling group (preferably, hydroxyl acid), and a compound having two or more hydroxy groups and a coupling group (preferably, diol compound). It is more preferably at least one selected from a group consisting of a compound having a carboxy group, an amino group, and a coupling group (preferably, an amino acid), a compound having a hydroxy group, an amino group, and a coupling group (preferably, an amino alcohol), and a compound having two or more carboxy groups and a coupling group (preferably, dicarboxylic acid).

Thus, from the viewpoint of controlling dissociation rate of a physiologically active substance from a polysaccharide derivative, the polysaccharide derivative of the second embodiment is preferably a compound which is composed of a combination of constitutional molecules selected from a group consisting of a combination of "a polysaccharide molecule having a carboxy group, a physiologically active substance having a hydroxyl group, and a spacer-forming molecule having an amino group and a carboxyl group", a combination of "a polysaccharide molecule having a carboxy group, a physiologically active substance having a hydroxyl group, a spacer-forming molecule having a hydroxyl group and a carboxyl group", a combination of "a polysaccharide molecule having a carboxy group, a physiologically active substance having a carboxy group, and a spacer-forming molecule having an amino group and a hydroxyl group", a combination of "a polysaccharide molecule having a carboxy group, a physiologically active substance having a carboxy group, and a spacer-forming molecule having two or more hydroxyl groups", a combination of "a polysaccharide molecule having an amino group, a physiologically active substance having a hydroxyl group, and a spacer-forming molecule having two or more carboxy groups", and a combination of "a polysaccharide molecule having an amino group, a physiologically active substance having a carboxy group, and a spacer-forming molecule having a carboxy group and a hydroxyl group. It is more preferably a compound which is composed of a combination of constitutional molecules selected from a group consisting of a combination of "a polysaccharide molecule having a carboxy group, a physiologically active substance having a hydroxyl group, and a spacer-forming molecule having an amino group and a carboxyl group", a combination of "a polysaccharide molecule having a carboxy group, a physiologically active substance having a carboxy group, and a spacer-forming molecule having an amino group and a hydroxyl group", a combination of "a polysaccharide molecule having an amino group, a physiologically active substance having a hydroxyl group, and a spacer-forming molecule having two or more carboxyl groups", and a combination of "a polysaccharide molecule having an amino group, a physiologically active substance having a carboxy group, and a spacer-forming molecule having a carboxy group and a hydroxyl group." It is still more preferably a compound which is composed of a combination of constitutional molecules selected from a group consisting of combinations of a combination of "a polysaccharide molecule having a carboxy group, a physiologically active substance having a hydroxyl group, and a spacer-forming molecule having an amino group and a carboxyl group, a combination of "a polysaccharide molecule having a carboxy group, a physiologically active substance having a carboxy group, and a spacer-forming molecule having an amino group and a hydroxyl group", and a combination of "a polysaccharide molecule having an amino group, a physiologically active substance having a hydroxyl group, and a spacer-forming molecule having at least two carboxy groups."

The coupling group contained in a spacer-forming molecule is not particularly limited if it is a divalent or a higher valent group which can couple by a covalent bond two or more reactive functional groups. Examples of the coupling group can include a divalent or a higher valent group derived from aliphatic hydrocarbons with 2 to 12 carbon atoms, a divalent or a higher valent group derived from aromatic hydrocarbons with 6 to 10 carbon atoms, and a combination thereof. The divalent or a higher valent group derived from aliphatic hydrocarbons means a hydrocarbon group which is formed by removing two or more hydrogen atoms from an aliphatic hydrocarbon molecule, and the site from which a hydrogen atom is removed is not particularly limited. The divalent or a higher valent group derived from aromatic hydrocarbons means an aromatic group which is formed by removing two or more hydrogen atoms from an aromatic hydrocarbon molecule, and the site from which a hydrogen atom is removed is not particularly limited.

From the viewpoint of suppressing antigenicity, the coupling group preferably has 12 or less atoms between reactive functional groups. It is more preferably 2 to 6 atoms.

The coupling group may have a substituent group. As the coupling group has a substituent group, the dissociation rate of a physiologically active substance in a polysaccharide derivative can be easily controlled to a desired range. Namely, without depending on a structure of a physiologically active substance, the dissociation rate of a physiologically active substance from a polysaccharide derivative can be controlled.

Site for a substituent group in the coupling group can be any site at which stability of the covalent bond between a group derived from a physiologically active substance and a spacer (preferably, ester bond) is affected and, in particular, it is preferably at least one site selected from a group consisting of $\alpha$ position and $\beta$ position of a reactive functional group which binds to a group derived from a physiologically active substance. Number of the substituent group can be selected depending on the purpose, and it is preferably 1 to 4, and more preferably 1 to 2. When there are two substituent groups, for example, the substitution position can be $\alpha$ position and $\beta$ position, or it may be $\alpha$ position only or $\beta$ position only.

Type of the substituent group on a coupling group is, for example, from the viewpoint of controlling dissociation rate of a physiologically active substance in a polysaccharide derivative, preferably at least one selected from a group consisting of an electron withdrawing group, an electron donating group, and a sterically hindered group.

For example, when a substituent group introduced to a coupling group is an electron withdrawing group, the covalent bond between a group derived from a physiologically active substance and a spacer decomposes more easily than a non-substitution case, and thus the dissociation rate of a physiologically active substance tends to increase. Thus, by selecting an electron withdrawing group based on electron withdrawing strength of an electron withdrawing group, for example, electronegativity, Hammett's constant, or the like as an indicator, it is possible to easily control the dissociation rate of a physiologically active substance to a desired range. Namely, there is a tendency that, by selecting a strong electron withdrawing group, the dissociation rate can be increased. Specifically, when a halogen atom like fluorine, chlorine, and bromine is introduced, the dissociation rate of a physiologically active substance tends to increase in the order of high electronegativity (F > Cl > Br).

The electron withdrawing group is not particularly limited as long as it can be introduced to a coupling group, and it can be suitably selected from generally used electron withdrawing groups. Specific examples of the electron withdrawing group can include a halogen atom like fluorine, chlorine, and bromine, an alkyl and aryl sulfoxide group, an alkyl and aryl sulfone group, a sulfonic acid group, an acetamide group, a carboxy group, an alkyl and arylcarbonyloxy group (ester group). Among them, it is preferably at least one selected from a group consisting of a halogen atom and an alkylcarbonyloxy group.

When the substituent group which is introduced to a coupling group is an electron donating group, the covalent bond between a group derived from a physiologically active substance and a spacer decomposes less easily than a non-substitution case, and thus the dissociation rate of a physiologically active substance tends to decrease. The electron donating group can be selected, for example, by using a Hammett's constant as an indicator.

The electron donating group is not particularly limited as long as it can be introduced to a coupling group, and it can be suitably selected from generally used electron donating groups. Specific examples of the electron donating group can include an alkene group and an alkyne group.

When the substituent group which is introduced to a coupling group is a sterically hindered group, the dissociation rate of a physiologically active substance tends to decrease. For a case in which the spacer-forming molecule is an amino acid, for example, the sterically hindered group can be selected by using the hydropathy index of an amino acid as an indicator.

Examples of the sterically hindered group can include a linear alkyl group such as a methyl group, an ethyl group, a propyl group, or a butyl group, a branched alkyl group such as an isopropyl group, an isobutyl group, or a t-butyl group, a cyclic alkyl group such as a cyclohexyl group, and an aryl group such as a phenyl group. Among them, a branched alkyl group, a cyclic alkyl group, and the like are preferable.

The substituent group on a coupling group may not be clearly classified into an electron withdrawing group, an electron donating group, or a sterically hindered group, but by selecting a substituent group and a substitution position thereof using a Hammett's constant, hydropathy index, or the like as an indicator, the dissociation rate can be controlled.

Furthermore, it is also possible that the dissociation rate is finely controlled by introducing both the electron withdrawing group and the electron donating group. Still furthermore, it is also possible that the dissociation rate is finely controlled by introducing a substituent group having an electron withdrawing or electron donating property and steric hindrance.

Controlling the dissociation rate of a physiologically active substance by use of a spacer can be combined with selection of a coupling site to a spacer in a physiologically active substance. Specifically, for example, when steroid is used as a physiologically active substance, the ester bond generated by a hydroxy group at position 11 is, in general, hardly hydrolyzed due to steric hindrance, and thus it is believed that the steroid as a physiologically active substance introduced to a polysaccharide derivative hardly dissociates. However, by suitably selecting a spacer which promotes hydrolysis of an ester bond from the aforementioned spacers, it becomes possible to dissociate the steroid from a polysaccharide derivative. In addition, compared to a case in which an ester bond is generated by a hydroxy group at position 21, for example, the dissociation duration is extended so that the continuous working duration of the steroid can be extended.

Namely, by selecting a structure of a spacer, the range of a physiologically active substance applicable to a polysaccharide derivative can be broadened.

Controlling the dissociation rate of a physiologically active substance by a use of a spacer can be combined with selection of molecular weight of a polysaccharide constituting the polysaccharide derivative.

The spacer-forming molecule is, although not particularly limited, preferably at least one selected from a group consisting of an amino acid, an amino alcohol, dicarboxylic acid, hydroxyl acid, a diol compound, and a derivative thereof. It is more preferably at least one selected from a group consisting of an amino acid, an amino alcohol, dicarboxylic acid, and a derivative thereof. It is still more preferably at least one selected from a group consisting of an amino acid, an amino alcohol, and a derivative thereof.

For a case in which the physiologically active substance has a hydroxy group, the spacer-forming molecule is preferably at least one selected from a group consisting of an amino acid, dicarboxylic acid, hydroxyl acid, and a derivative thereof. It is more preferably at least one selected from a group consisting of an amino acid, dicarboxylic acid, and a derivative thereof. It is still more preferably at least one selected from a group consisting of an amino acid and a derivative thereof.

For a case in which the physiologically active substance has a carboxy group, the spacer-forming molecule is preferably at least one selected from a group consisting of an amino alcohol, hydroxyl acid, a diol compound, and a derivative thereof. It is more preferably at least one selected from a group consisting of an amino alcohol and a derivative thereof.

The amino acid as a spacer-forming molecule is not particularly limited if it is a compound having an amino group and a carboxy group. It may be either an amino acid of natural origin or a synthetic amino acid.

Specific examples of the amino acid can include glycine, alanine, β-alanine, arginine, asparagine, serine, asparaginic acid, cysteine, glutamine, glutamic acid, proline, tyrosine, tryptophan, lysine, methionine, phenylalanine, threonine, valine, isoleucine, leucine, histidine, norvaline, norleucine, isoserine, ornithine, aminobutyric acid, aminovaleric acid, aminoheptanoic acid, aminooctanoic acid, aminodecanoic acid, aminoundecanoic acid, aminododecanoic acid, and a structural isomer thereof.

Among them, ω-aminofatty acid like glycine, β-alanine, aminobutyric acid, aminovaleric acid, aminoheptanoic acid, aminooctanoic acid, aminodecanoic acid, aminoundecanoic acid, and aminododecanoic acid can be preferably used. Meanwhile, those ω-aminofatty acids may have a substituent group, and it is preferable to have a halogen atom on α position or β position of a carboxy group. Number of the carbon atom in ω-aminofatty acid is not particularly limited, but from the viewpoint of controlling the dissociation rate, it is preferably 3 or more, more preferably 3 to 12, and still more preferably 3 to 6. Meanwhile, the number of carbon atoms in ω-aminofatty acid indicates the total carbon number including the carbonyl carbon of a carboxy group.

Examples of the amino acid derivative include a halogenated derivative in which the hydrogen atom of the carbon at α position, which is adjacent to the carboxy group, is substituted with a halogen, e.g., 2-fluoro-3-amino-propanoic acid, 2-chloro-3-amino-propanoic acid, and 2-bromo-3-amino-propanoic acid, an alkyl substituted derivative in which the hydrogen atom of the carbon at α position, which is adjacent to the carboxy group, is substituted with an alkyl chain, e.g., 2-methyl-2-aminopropanoic acid, and diethylglycine, and an amino acid derivative with an ether bond like 11-amino-3,6,9-trioxaundecanoic acid.

As for the amino acid derivative, a derivative selected from the aforementioned amino acids, or a dipeptide, a tripeptide, or a tetrapeptide resulting from a combination thereof can be also used.

Specific examples of the amino alcohol can include aliphatic amino alcohol such as aminoethanol, aminopropanol, aminobutanol, or aminohexanol; and aminoarylalkylalcohol such as aminophenylethanol or aminobenzylalcohol; and a structural isomer thereof.

Examples of the amino alcohol derivative can include halogenated amino alcohol in which the hydrogen atom of the carbon at $\alpha$ position or the carbon at $\beta$ position, which is adjacent to the first position or second position when counted from the hydroxy group, such as 3-amino-2-fluoro-1-propanol or 4-amino-3-fluoro-1-butanol.

Specific examples of the dicarboxylic acid can include aliphatic dicarboxylic acid such as oxalic acid, malonic acid, succinic acid, glutaric acid, adipic acid, pimellic acid, suberic acid, azellaic acid, or sebacic acid; aromatic dicarboxylic acid such as phthalic acid, isophthalic acid, or terephthalic acid; and a structural isomer thereof.

Examples of the dicarboxylic acid derivative include tartronic acid, tartaric acid, isocitric acid, citric acid, citramalic acid, and malic acid.

Examples of the hydroxyl acid can include glycolic acid, lactic acid, 2-hydroxybutyric acid, 3-hydroxybutyric acid, $\gamma$-hydroxybutyric acid, 2-phosphoglyserine acid, 3-phosphoglyserine acid, leucine acid, ricinoleic acid, cereblonic acid, and a structural isomer thereof.

Examples of the hydroxyl acid derivative include malic acid, glycerin acid, tartronic acid, tartaric acid, pantoic acid, and mevalonic acid.

The spacer in a polysaccharide derivative is, from the viewpoint of controlling the dissociation ratio of a physiologically active substance from a polysaccharide derivative, preferably a divalent or higher valent group which is derived from at least one selected from a group consisting of an amino acid, an amino alcohol, dicarboxylic acid, and a derivative thereof. It is more preferably a divalent or higher valent group which is derived from at least one selected from a group consisting of an amino acid, an amino alcohol, and a derivative thereof. It is still more preferably a divalent or higher valent group which is derived from at least one selected from a group consisting of an amino acid and a derivative thereof.

When the spacer in a polysaccharide derivative is a divalent or higher valent group derived from an amino acid or a derivative thereof, the spacer is preferably a divalent group derived from an amino acid that is represented by the following general formula (I). The spacer represented by the general formula (I) can be preferably applied for a case in which the physiologically active substance has a hydroxy group.

$$-HN-(CH_2)_m-CR^{11}R^{12}-CR^{13}R^{14}-CO- \qquad (I)$$

In the formula, m is an integer of 0 to 12. $R^{11}$, $R^{12}$, $R^{13}$, and $R^{14}$ each independently represent a hydrogen atom, an electron withdrawing group, an electron donating group, or a sterically hindered group. m is preferably 0 to 8, and more preferably 0 to 4. Furthermore, $R^{11}$, $R^{12}$, $R^{13}$, and $R^{14}$ groups may be the same or different from each other, either completely or partially.

Details of the electron withdrawing group, electron donating group, and sterically hindered group are as defined above. By suitably selecting $R^{11}$, $R^{12}$, $R^{13}$, and $R^{14}$ in the spacer represented by the general formula (I), the dissociation rate of a physiologically active substance from a polysaccharide derivative can be controlled. Thus, at least one of $R^{11}$ to $R^{14}$ is preferably an electron withdrawing group, an electron donating group, or a sterically hindered group.

For example, when at least one of $R^1$ to $R^{14}$ is an electron withdrawing group, the coupling strength is lowered so that the physiologically active substance is more easily dissociated and the dissociation rate is increased.

In terms of the influence of an ester bond on the dissociation rate, a higher effect can be obtained when an electron withdrawing group is introduced to $R^{13}$ or $R^{14}$ instead of $R^{11}$ and $R^{12}$. Furthermore, by selecting an electron withdrawing group in accordance with the strength of an electron withdrawing property, e.g., electronegativity, it becomes possible to control the dissociation rate of a physiologically active substance. Namely, in general, there is a tendency that the dissociation rate increases by introducing a substituent group with higher electronegativity.

The electron withdrawing group introduced to $R^{11}$ to $R^{14}$ can be any kind of an electron withdrawing group as long as it can be introduced, and examples thereof include a halogen atom, an alkyl and aryl sulfoxide group, an alkyl and aryl sulfone group, a sulfonic acid group, an acetamide group, a carboxy group, and an alkyloxy carbonyl group.

For example, when a halogen atom like fluorine, chlorine, and bromine is introduced to $R^{13}$ or $R^{14}$, the dissociation rate of a physiologically active substance tends to increase in the order of high electronegativity (F > Cl > Br) due to the reason described above.

On the contrary, when an electron donating group is introduced to $R^{13}$ or $R^{14}$, it is possible to lower the dissociation rate of a physiologically active substance. The electron donating group for $R^{13}$ or $R^{14}$ is not particularly limited as long as it has a structure capable of supplying an electron.

In order to further lower the dissociation rate of a physiologically active substance, it is possible to cause steric hindrance by introducing to a spacer a linear alkyl group such as a methyl group, an ethyl group, a propyl group, or a butyl group, a branched alkyl group such as an isopropyl group, an isobutyl group, or a t-butyl group, a cyclic alkyl group such as a cyclohexyl group, and an aryl group such as a phenyl group, and thus lowering the dissociation rate.

It is also possible that, by having $R^{11}$ (or $R^{12}$), and $R^{13}$ (or $R^{14}$) as an electron withdrawing group and an electron donating group, respectively, the dissociation rate is finely controlled. Furthermore, by having a substituent group having an electron withdrawing property or an electron donating property with steric hindrance as $R^{11}$ to $R^{14}$, the dissociation rate can be finely controlled.

Specific examples of the spacer represented by the general formula (I) can include spacers described in the following Table 1, but this embodiment is not limited to them. Meanwhile, in the following Table 1, H represents a hydrogen atom, F represents a fluorine atom, Cl represents a chlorine atom, Br represents a bromine atom, OH represents a hydroxy group, and OTs represents a toluenesulfonyloxy group.

[Table 1]

|  | m | $R^{11}$ | $R^{12}$ | $R^{13}$ | $R^{14}$ |
|---|---|---|---|---|---|
| I-1 | 0 | H | H | H | H |
| I-2 | 0 | H | H | F | H |
| I-3 | 0 | H | H | Cl | H |
| I-4 | 0 | H | H | Br | H |
| I-5 | 0 | H | H | OH | H |
| I-6 | 0 | H | H | 0Ts | H |

When the spacer in a polysaccharide derivative is a divalent or higher valent group derived from an amino alcohol or a derivative thereof, the spacer is preferably a divalent group derived from an amino alcohol that is represented by the following general formula (II). The spacer represented by the general formula (II) can be preferably applied for a case in which the physiologically active substance has a carboxy group.

$$-HN-(CH_2)_n-CR^{21}R^{22}-CR^{23}R^{24}-O- \qquad (II)$$

In the formula, n is an integer of 0 to 12. $R^{21}$, $R^{22}$, $R^{23}$, and $R^{24}$ each independently represent a hydrogen atom, an electron withdrawing group, an electron donating group, or a sterically hindered group. n is preferably 0 to 8, and even more preferably 0 to 4. Furthermore, $R^{21}$, $R^{22}$, $R^{23}$, and $R^{24}$ groups may be the same or different from each other, either completely or partially.

Details of the electron withdrawing group, electron donating group, and sterically hindered group are as defined above. By suitably selecting $R^{21}$, $R^{22}$, $R^{23}$, and $R^{24}$ in the spacer represented by the general formula (II), the dissociation rate of a physiologically active substance from a polysaccharide derivative can be controlled. Thus, at least one of $R^{21}$ to $R^{24}$ is preferably an electron withdrawing group, an electron donating group, or a sterically hindered group.

For example, when a spacer in which $R^{21}$ or $R^{22}$ is an electron withdrawing group is used, the dissociation rate of a physiologically active substance is increased. The electron withdrawing group introduced to $R^{21}$ or $R^{22}$ can be any kind of an electron withdrawing group as long as it can be introduced, and examples thereof include a halogen atom, an alkyl and aryl sulfoxide group, an alkyl and aryl sulfone group, a sulfonic acid group, an acetamide group, a carboxy group, and an alkyloxy carbonyl group.

For example, a physiologically active substance in a derivative in which $R^{21}$ is a halogen atom with high electronegativity and $R^{22}$ is a hydrogen atom has a high dissociation rate. Furthermore, by having a halogen atom for both of $R^{21}$ and $R^{22}$, the dissociation rate further increases. Furthermore, when $R^{23}$ or $R^{24}$ closer to an ester bond is an electron withdrawing group, it is also possible to further accelerate the dissociation rate of a physiologically active substance.

On the contrary, when $R^{21}$ or $R^{22}$ is an electron donating group, the dissociation rate of a physiologically active substance can be further reduced. The electron donating group as $R^{21}$ or $R^{22}$ is not particularly limited as long as it has a structure capable of supplying an electron.

When an alkyl group or the like is introduced to $R^{23}$ or $R^{24}$ and the hydroxy group of a spacer-forming molecule is a secondary or tertiary hydroxy group, the dissociation rate is reduced. It is also possible to reduce the dissociation rate by causing steric hindrance according to introduction of a linear alkyl group such as a methyl group, an ethyl group, a propyl group, or a butyl group, a branched alkyl group such as an isopropyl group, an isobutyl group, or a t-butyl group, a cyclic alkyl group such as a cyclohexyl group, and an aryl group such as a phenyl group to $R^{23}$ or $R^{24}$.

By having two selected from $R^{21}$ to $R^{24}$ as an electron withdrawing group and an electron donating group, the dissociation rate can be finely controlled. In addition, by having $R^{21}$ to $R^{24}$ as a substituent group which has an electron withdrawing property or an electron donating property with steric hindrance, the dissociation rate can be also finely controlled.

Specific examples of the spacer represented by the general formula (II) can include spacers described in the following Table 2, but this embodiment is not limited to them. Meanwhile, in the following Table 2, H represents a hydrogen atom, F represents a fluorine atom, Cl represents a chlorine atom, Br represents a bromine atom, Me represents a methyl group, and COOEt represents an ethoxycarbonyl group.

[Table 2]

|  | n | R²¹ | R²² | R²³ | R²⁴ |
|---|---|---|---|---|---|
| II-1 | 1 | H | H | H | H |
| II-2 | 1 | F | H | H | H |
| II-3 | 1 | F | F | H | H |
| II-4 | 1 | Cl | H | H | H |
| II-5 | 0 | COOEt | H | H | H |
| II-6 | 0 | H | H | Me | H |
| II-7 | 0 | COOEt | H | Me | H |

The polysaccharide derivative is preferably a polysaccharide derivative in which a group derived from a polysaccharide is covalently bonded with at least one group derived from a physiologically active substance, which is selected from a group consisting of a steroid, a xanthine derivative, an anti-allergic drug, and an anti-choline drug, via a divalent or higher valent group which is derived from a compound selected from a group consisting of $\omega$-aminofatty acid with 3 or more carbon atoms, an amino alcohol, dicaboxylic acid, and a derivative thereof. The polysaccharide is preferably at least one selected from a group consisting of glycosaminoglycan and homoglycan. The polysaccharide is more preferably at least one selected from a group consisting of hyaluronic acid, chondroitin sulfate, chitosan, and carboxymethyl cellulose

Furthermore, it is particularly preferable that, in the polysaccharide derivative, a group derived from a polysaccharide, which is chondroitin sulfate, is covalently bonded to a group derived from a physiologically active substance, which is a steroid, via a divalent or higher valent group derived from $\omega$-aminofatty acid with 3 or more carbon atoms or a derivative thereof.

As for the steroid, it is preferably at least one selected from a group consisting of betamethasone, prednisolone, budesonide, and fluticasone.

The $\omega$-aminofatty acid with 3 or more carbon atoms is preferably at least one selected from a group consisting of $\beta$-alanine and 4-aminobutyric acid. It is more preferably $\beta$-alanine.

As for the $\omega$-aminofatty acid derivative, a derivative in which a halogen atom is substituted with carbon on $\alpha$ position of the carboxy group is preferable.

The ratio between a content number of a constructional unit composed of a disaccharide which constitutes a group derived from a polysaccharide and a content number of a group derived from a physiologically active substance in the polysaccharide derivative is not particularly limited. The ratio can be represented by the mole number ratio of a group derived from an introduced physiologically active substance relative to total mole number of a constructional disaccharide unit in the polysaccharide (hereinbelow, it may be expressed as introduction ratio). The introduction ratio is, although not particularly limited, 1% to 100%, for example.

Meanwhile, the content ratio of a physiologically active substance can be suitably selected depending on use, type, and activity strength, a property of a physiologically active substance like solubility in water, and type of a spacer, or the like. For example, it is preferably prepared such that the content of a physiologically active substance is 1 $\mu$g to 100 mg per single administration.

The polysaccharide derivative may be composed of a combination of a single type of polysaccharide molecule and a single type of physiologically active substance. It may be also a combination of a single type of polysaccharide molecule and two or more types of physiologically active substance, or a combination of two or more types of polysaccharide molecule and a single type of physiologically active substance.

The dissociation rate of a physiologically active substance from a polysaccharide derivative is not particularly limited, and it can be suitably selected depending on the purpose or the like. The dissociation rate of a physiologically active substance from a polysaccharide derivative is, in phosphate buffered saline at pH 7.5, 36°C, for example, preferably 0.1 to 30%/day, more preferably 0.3 to 10%/day, and even more preferably 0.5 to 5%/day. Herein, the dissociation rate of a physiologically active substance, which corresponds to a variation amount of dissociation ratio (%) per day, is defined by slope of a graph in which time is plotted on a horizontal axis and the dissociation ratio (%), which is a ratio of the dissociation amount (mole) of a physiologically active substance relative to total mole number (100%) of a group derived from a physiologically active substance contained in the polysaccharide derivative, is plotted on a vertical axis. Thus, when the dissociation rate maintains a constant value during the measurement period, the graph shows a monotonically increasing straight line, and for a case in which all the physiologically active substances contained in a polysaccharide derivative are dissociated within 10 days in a 10 mM phosphate buffered saline at pH 7.5, 36°C, the dissociation rate is expressed as 10%/day. Meanwhile, when the dissociation rate shows a decrease over the time instead of having a constant value (i.e., graph shows a mild curve having a bulge in the upward direction and monotonic increase), the change in dissociation ratio during initial increase period (e.g., for 3 days) is approximated to a straight line to calculate the dissociation rate.

Meanwhile, the dissociation amount of a physiologically active substance can be measured by a method which is suitably selected depending on the type of a physiologically active substance.

(Method for producing polysaccharide derivative)

The polysaccharide derivative can be produced by forming a covalent bond between a functional group of a polysaccharide molecule (e.g., carboxy group, amino group, or hydroxyl group) and a functional group of a physiologically active substance (e.g., carboxy group or hydroxyl group) either directly or with a spacer therebetween.

Hereinbelow, as an exemplary method for producing a polysaccharide derivative, explanations are given for a method for producing a polysaccharide derivative in which a group derived from a physiologically active substance is linked to a group derived from a polysaccharide via a covalent bond including an ester bond.

When a polysaccharide molecule and a physiologically active substance are directly linked via a covalent bond, as the carboxy group or hydroxyl group in a polysaccharide molecule and a hydroxyl group or a carboxy group in a physiologically active substance are covalently bonded to each other by a commonly used esterification method, a polysaccharide derivative can be produced. As a method for esterification, a method of using a condensation agent such as water soluble carbodiimide (e.g., 1-ethyl-3-(3-dimethylaminopropyl)carbodiimide) or DMT-MM (4-(4,6-dimethoxy-1,3,5-triazin-2-yl)-4-methylinorpholinium chloride n-hydrate), a symmetric acid anhydride method, a mixed acid anhydride method, and an active esterification can be mentioned. The reaction conditions for esterification method can be suitably selected depending on an esterification method for application.

When a polysaccharide molecule and a physiologically active substance are covalently bonded to each other with a spacer therebetween, it can be produced by the production method including the following steps, for example.

(1a) Forming an ester bond between a carboxy group or a hydroxyl group of a physiologically active substance and a reactive functional group of a spacer-forming molecule, and
(2a) Forming a covalent bond according to condensation between a carboxy group or an amino group of a polysaccharide molecule and a reactive functional group of a spacer-forming molecule.

According to the step (1a), an ester bond is formed, by a commonly used esterification method, between a carboxy group or a hydroxyl group of a physiologically active substance and a reactive functional group of a spacer-forming molecule (preferably, hydroxyl group or carboxy group). At that time, the reactive functional group of a spacer-forming molecule expected to be bonded to the polysaccharide may be protected in advance by a commonly used method, if necessary.

According to the step (2a), a covalent bond is formed according to condensation between a carboxy group or an amino group of a polysaccharide molecule and a reactive functional group of a spacer-forming molecule (preferably, amino group or carboxy group). The condensation method can be suitably selected from a commonly used method, depending on a covalent bond to be formed. At that time, the reactive functional group of a spacer-forming molecule expected to be bonded to the physiologically active substance may be protected in advance by a commonly used method, if necessary.

Furthermore, from the viewpoint of controlling the dissociation rate of a physiologically active substance from a polysaccharide derivative, it is preferable that the polysaccharide derivative is produced by the production method including the following steps of:

(1b) preparing a physiologically active substance having a carboxy group or a hydroxy group,
(2b) selecting a spacer for forming a covalent bond with a group derived from a physiologically active substance in accordance with decomposition rate of the covalent bond, and
(3b) forming a covalent bond between the group derived from a physiologically active substance and a group derived from polysaccharide with the spacer therebetween.

According to the step (1b), a desired physiologically active substance can be suitably prepared from a known physiologically active substance. Alternatively, a desired physiologically active substance can be produced by a known method.

According to the step (2b), a spacer-forming molecule is selected such that a desired dissociation rate of a physiologically active substance can be obtained. The spacer-forming molecule has a hydroxyl group or carboxy group as a reactive functional group for forming an ester bond with a physiologically active substance, a reactive functional group (preferably, amino group or carboxy group) for forming a covalent group (preferably amide bond) with polysaccharide, and a coupling group which is selected such that it can couple the two reactive functional groups and control the dissociation rate of an ester bond to a physiologically active substance. Details for selecting the spacer-forming molecules are as described above.

According to the step (3b), a polysaccharide derivative is produced by using a prepared physiologically active substance, a selected spacer-forming molecule, and polysaccharide. For the step (3b), the aforementioned method for producing a polysaccharide derivative can be applied, for example.

The pharmaceutical composition of this embodiment may contain only one type of a polysaccharide derivative or two or more of them in combination. When two or more kinds of a polysaccharide derivative are contained, it can be a combination of two or more different kinds of polysaccharide derivatives in which two or more different kinds of polysaccharides are combined with one physiologically active substance or a combination of two or more different kinds of polysaccharide derivatives in which two or more kinds of physiologically active substances are combined with one polysaccharide.

Content of the polysaccharide derivative in a pharmaceutical composition is not particularly limited, and it can be suitably selected depending on the purpose or the like.

The pharmaceutical composition may contain, in addition to a polysaccharide derivative, other components including pharmaceutically acceptable vehicle or the like. Examples of other components can include a surface active agent, a physiologically active substance, a stabilizer, and a liquid medium as well as a pharmaceutically acceptable vehicle or the like. The physiologically active substance may be the same or different from a physiologically active substance which is dissociated from the polysaccharide derivative.

The pharmaceutical composition of this embodiment is preferably used for administration to a respiratory organ. Specific examples of the administration to a respiratory organ include intrapulmonary administration, intranasal administration, or the like.

Preferably, the pharmaceutical composition may be in the form administrable to a respiratory organ, and it may be any form like powder, liquid, and suspension.

Examples of administration methods can include an administration method using inhalation by a user himself, air stream created by an external device, or a method of using both of them, and an administration method using a nebulizer.

The pharmaceutical composition administered to a respiratory organ is arrived and adhered to a target tissue of a respiratory organ while a physiologically active substance and a polysaccharide are still covalently bonded to each other. After that, as the covalent bond between the spacer and the physiologically active substance is gradually decomposed, sustained release of a physiologically active substance is achieved for an extended period of time (e.g., 24 hours or longer).

Examples of the target tissue include a pulmonary tissue (including alveolus, terminal bronchiole, bronchiole, and bronchus), nasal cavity (including paranasal cavity, frontal sinus, ethmoid sinus, maxillary sinus, sphenoidal sinus, superior turbinate, middle turbinate, and inferior turbinate).

In a target tissue, a physiologically active substance can be gradually dissociated from the pharmaceutical composition. The time during which a physiologically active substance is continuously dissociated in a target tissue (i.e., sustaining property of the effect) is preferably 72 hours or longer, and more preferably 120 hours (5 days) or longer.

The pharmaceutical composition is preferably a pharmaceutical composition which can continuously dissociate a physiologically active substance for 72 hours or longer. It is more preferably a pharmaceutical composition which can continuously dissociate it for 120 hours (5 days) or longer.

The long-acting effect of the pharmaceutical composition in a target tissue can be evaluated in accordance with a target tissue.

For example, when the target tissue is a lung, pH of an alveolar lining layer is generally known to be weakly alkaline (i.e., pH of 7.5 or so). Thus, when a pharmaceutical composition as a subject for evolution is dissolved in 10 mM phosphate buffer solution prepared to have pH of about 7.5 and stored for 1 week at 36°C, the long-acting effect can be evaluated based on the dissociation ratio of a physiologically active substance.

Furthermore, when the target tissue is a nasal cavity, pH of nasal cavity mucosa from a patient suffering from allergic rhinitis is generally known to be weakly alkaline (i.e., pH of 8.0 or so). Thus, when a pharmaceutical composition as a subject for evolution is dissolved in 10 mM borate buffer solution prepared to have pH of about 8.0 and stored for 1 week at 36°C, the long-acting effect can be evaluated based on the dissociation ratio of a physiologically active substance.

The pharmaceutical composition can be suitably applied to therapeutics of a respiratory disorder, for example, but the use of the pharmaceutical composition is not limited to therapeutics of a respiratory disorder. It can be applied for, in addition to prevention of a respiratory disorder, treatment, prevention, diagnosis, or the like of other disorders, depending on the type of a physiologically active substance.

When the pharmaceutical composition is used for therapeutics or prophylaxis of a respiratory disorder, examples of the respiratory disorder include bronchial asthma, chronic obstructive pulmonary disorder (COPD), viral infection, acute allergic disorder, and chronic allergic disorder.

EXAMPLES

Hereinbelow, the present invention is explained in greater detail by referring to Examples and Test examples, but it is evident that the technical scope of the present invention is not limited to them. Meanwhile, except the dissociation ratio and introduction ratio, "%" is based on mass, unless specifically described otherwise.

(Example 1) Preparation of chondroitin sulfate introduced with 21-β-alanyl-betamethasone

1-1. Preparation of 21-(Boc-β-alanyl)-betamethasone

2 g (10.6 mmol) of Boc-β-alanine was added with 20 mL of dichloromethane, 20 mL of dimethyl formamide, 4.15 g of betamethasone, and 388 mg of N,N-dimethyl-4-aminopyridine. After that, under ice cooling, 2.63 g of water soluble carbodiimide (manufactured by WATANABE CHEMICAL INDUSTRIES, LTD) was added and stirred overnight at room temperature. After confirming by thin layer chromatography the disappearance of the reacting materials, a saturated aqueous solution of ammonium chloride was added under ice cooling and liquid fractionation extraction was performed 3 times by using dichloromethane and water. The collected organic layer was washed in order with a saturated aqueous solution of ammonium chloride, a saturated aqueous solution of sodium hydrogen carbonate, and a saturated aqueous brine solution. After drying over magnesium sulfate, it was concentrated under reduced pressure by using an evaporator in water bath at 40°C, and as a result, 6.01 g of the desired compound 1 was obtained (yield: quant.). Meanwhile, Boc is an abbreviation of a tert-butoxycarbonyl group.

1-2. Preparation of 21-β-alanyl-betamethasone

6.01 g of the compound 1 was dissolved in 30 mL of dichloromethane and 65 mL of tetrahydrofuran, and under ice cooling, added with 90 mL of 4 M hydrochloric acid/ethyl acetate and stirred for 4 hours. The reaction solution was concentrated under reduced pressure by using an evaporator in water bath at 40°C. As a result, the desired compound 2 was obtained in an amount of 4.26 g (yield of 80%).

1-3. Preparation of chondroitin sulfate introduced with 21-β-alanyl-betamethasone

3 g of Sodium chondroitin sulfate (weight average molecular weight of 25 kDa) was dissolved in 200 mL of distilled water and 200 mL of ethanol, and after being added with 597 mg of the compound 2 and 560 mg of 4-(4,6-dimethoxy-1,3,5-triazin-2-yl)-4-methylmorpholinium chloride n-hydrate (DMT-MM), it was stirred overnight. After that, it was added with 2.25 g of sodium hydrogen carbonate followed by stirring for 3 hours. Then, 1.2 mL of acetic acid, 9 g of sodium chloride, and 600 mL of 90% ethanol/distilled water were added in order for forming precipitates. The supernatant was discarded and washing with 90% ethanol/distilled water was performed 2 times. The obtained precipitates were dried overnight under reduced pressure, and as a polysaccharide derivative introduced with betamethasone as a pharmaceutical, 2.9 g of the desired compound ia was obtained. As a result of obtaining the introduction ratio by $^1$H-NMR, it was found to be about 18%.
Meanwhile, the introduction ratio of a pharmaceutical is based on mole, and it remains the same in the following.

1-4. Preparation of chondroitin sulfate introduced with high amount of 21-β-alanyl-betamethasone

2 g of Sodium chondroitin sulfate (weight average molecular weight of 20 kDa) was dissolved in 25 mL of distilled water and 25 mL of ethanol, and after being added with 1491 mg of the compound 2 and 1398 mg of 4-(4,6-dimethoxy-1,3,5-triazin-2-yl)-4-methylmorpholinium chloride n-hydrate, it was stirred overnight. After that, it was added with 2 g of sodium chloride and 150 mL of 90% ethanol/distilled water in order for forming precipitates. The supernatant was discarded and washing with 90% ethanol/distilled water was performed 2 times. The obtained precipitates were dried overnight under reduced pressure, and as a polysaccharide derivative introduced with betamethasone as a pharmaceutical, 2.9 g of the desired compound ib was obtained. As a result of obtaining the introduction ratio by $^1$H-NMR, it was found to be about 66%.

(Example 2) Preparation of chondroitin sulfate introduced with 21-(2-fluoro-β-alanyl)-betamethasone

2-1. Preparation of Boc-2-fluoro-β-alanine

600 mg (4.18 mmol) of a 2-fluoro-β-alanine hydrochloride salt was dissolved in 24 mL of tert-butanol and 12 mL of 1 M sodium hydroxide solution, and under ice cooling, added with 1.19 g of di-tert-butyl bicarbonate. After that, it was stirred overnight at room temperature and it was concentrated under reduced pressure by using an evaporator in water bath at 40°C. Then, liquid fractionation extraction was performed using ethyl acetate and 1 M hydrochloric acid, and the collected organic layer was washed with a saturated aqueous brine solution. After drying over magnesium sulfate, it was concentrated under reduced pressure by using an evaporator at 40°C. As a result, the desired compound 3 was obtained in an amount of 865 mg (yield of 99%).
$^1$H-NMR(500MHz,CDCl$_3$)δ1.45(9H,s,Boc),3.64-3.79(2H,br),4.98(1H,br),5.05(1H,br.a mide)

2-2. Preparation of 21-(Boc-2-fluoro-β-alanyl)-betamethasone

500 mg (2.41 mmol) of the compound 3 was dissolved in 30 mL of dimethyl formamide, and added with 947 mg of betamethasone and 89 mg of N,N-dimethyl-4-aminopyridine. After that, under ice cooling, 694 mg of water soluble carbodiimide was added and stirred overnight at room temperature. After confirming by thin layer chromatography the disappearance of the reacting materials, liquid fractionation extraction was performed 3 times by using toluene and water, and the collected organic layer was washed in order with a saturated aqueous solution of ammonium chloride, a saturated aqueous solution of sodium hydrogen carbonate, and a saturated aqueous brine solution. After drying over magnesium sulfate, it was concentrated under reduced pressure by using an evaporator in water bath at 60°C, and the desired compound 4 was obtained in an amount of 1.4 g (yield of 99%).
$^1$H-NMR(500MHz,CDCl$_3$)
δ1.04(3H,d),1.16(3H,m),1.17(3H,m),1.46(9H,s,Boc),1.84(2H,m),1.86(2H,m),2.01(2H, m),2.31(2H,m),2.34(1H,m),2.40(1H,m),2.65(1H,dt),3.60-3.72(2H,br),3.89-4.00(1H,m), 4.41(1H,d),4.95(1H,t),5.15(1H,br,amide),6.12(1H,s),6.35(d),7.21(1H,d)

2-3. Preparation of 21-(2-fluoro-β-alanyl)-betamethasone

250 mg of the compound 4 was dissolved in 20 mL of dichloromethane, and under ice cooling, added with 16 mL of 4 M hydrochloric acid/ethyl acetate, and stirred for 3 hours. After confirming by thin layer chromatography the disappearance of the reacting materials, it was concentrated under reduced pressure by using an evaporator in water bath at 40°C. As a result, the desired compound 5 was obtained in an amount of 188 mg (yield of 82%).
$^1$H-NMR(500MHz,CD$_3$OD)
δ1.04(3H,d),1.12(3H,m),1.21(3H,m),1.54(2H,m),1.60(2H,m),2.01(2H,m),2.31 (2H,m),2. 34(1H,m),2.40(1H,m),2.65(1H,dt),3.60-3.72(2H,m),4.20(1H,m),5.01(1H,m),5.35(1H,m ),6.02(1H,s),6.28(d),7.33(1H,d)

2-4. Preparation of chondroitin sulfate introduced with 21-(2-fluoro-β-alanyl)-betamethasone

1 g of sodium chondroitin sulfate (weight average molecular weight of 25 kDa) was dissolved in 80 mL of distilled water and 80 mL of ethanol followed by addition of 349 mg of the compound 5. After that, 317 mg of 4-(4,6-dimethoxy-1,3,5-triazin-2-yl)-4-methylinorpholinium chloride n-hydrate was added thereto and it was stirred overnight. After that, it was added with 3 g of sodium chloride followed by stirring for 30 minutes, and 160 mL of 90% ethanol/distilled water was added for forming precipitates. The obtained precipitates were dried overnight under reduced pressure, and as a polysaccharide derivative, 1.08 g of the desired compound ii was obtained. As a result of obtaining the introduction ratio by a carbazole sulfate method, it was found to be 16% in terms of molar basis concentration relative to entire carboxyl groups in the chondroitin sulfate.

(Example 3) Preparation of hyaluronic acid introduced with 21-β-alanyl-betamethasone

3-1. Preparation of hyaluronic acid introduced with 21-β-alanyl-betamethanone (880 kDa)

1 g of sodium hyaluronate (weight average molecular weight of 880 kDa) was dissolved in 100 mL of water for injection (WFI) and 100 mL of EtOH and added with 249 mg of the compound 2. After that, 234 mg of 4-(4,6-dimethoxy-1,3,5-triazin-2-yl)-4-methylmorpholinium chloride n-hydrate was added thereto and stirred overnight. Then, 750 mg of sodium hydrogen carbonate was added, stirred for 4 hours, and added in order with 400 μL of acetic acid and 3 g of sodium chloride. After stirring for 30 minutes, 200 mL of 90% ethanol/distilled water was added to form precipitates. The supernatant was discarded and washing with 90% ethanol/distilled water was performed 2 times. The obtained precipitates were dried overnight under reduced pressure, and as a polysaccharide derivative, 1.06 g of the desired compound iiia was obtained. As a result of obtaining the introduction ratio by a carbazole sulfate method, it was found to be 16%.

3-2. Preparation of hyaluronic acid introduced with 21-β-alanyl-betamethasone (490 kDa)

To 1 g of sodium hyaluronate (weight average molecular weight of 490 kDa), 100 mL of water for injection was added and stirred overnight. 100 mL of ethanol was slowly added and the solution was homogenously stirred. Thereafter, 231.3 mg of the compound 2 was added thereto. Subsequently, 138.4 mg of 4-(4,6-dimethoxy-1,3,5-triazin-2-yl)-4-methylmorpholinium chloride n-hydrate was added thereto followed by stirring overnight. Then, 750 mg of sodium hydrogen carbonate was added followed by stirring for 3 hours and neutralization was performed according to addition of 0.2 mL of acetic acid. After adding 1 g of sodium chloride, the reaction solution was added with 230 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at 40°C under reduced pressure conditions (75 mmHg), the desired compound iiib (0.90 g) was obtained as a polysaccharide derivative. As a result of measuring $^1$H-NMR, the introduction ratio of betamethasone in the compound was found to be 20%.

3-3. Preparation of hyaluronic acid introduced with 21-β-alanyl-betamethasone (270 kDa)

To 1 g of sodium hyaluronate (weight average molecular weight of 270 kDa), 100 mL of water for injection was added and stirred overnight. 100 mL of ethanol was slowly added and the solution was homogenously stirred. Thereafter, 231.3 mg of the compound 2 was added thereto. Subsequently, 138.4 mg of 4-(4,6-dimethoxy-1,3,5-triazin-2-yl)-4-methylmorpholinium chloride n-hydrate was added thereto followed by stirring overnight. Then, 750 mg of sodium hydrogen carbonate was added followed by stirring for 3 hours and neutralization was performed according to addition of 0.2 mL of acetic acid. After adding 1 g of sodium chloride, the reaction solution was added with 230 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at 40°C under reduced pressure conditions (75 mmHg), the desired compound iiic (0.95 g) was obtained as a polysaccharide derivative. As a result of measuring $^1$H-NMR, the introduction ratio of betamethasone in the compound was found to be 18%.

(Example 4) Preparation of chondroitin sulfate introduced with 21-β-alanyl-betamethasone (10 kDa)

To 1 g of sodium chondroitin sulfate (weight average molecular weight of 10 kDa), 15 mL of water for injection was added and stirred for 30 minutes to dissolve it. 15 mL of ethanol was slowly added and the solution was homogenously stirred. Thereafter, 185.0 mg of the compound 2 was dissolved in 5 mL solution of ethanol/water for injection =1/1 and added thereto. Subsequently, 110.7 mg of 4-(4,6-dimethoxy-1,3,5-triazin-2-yl)-4-methylmorpholinium chloride n-hydrate was dissolved in 5 mL solution of ethanol/water for injection = 1/1 and added thereto followed by stirring overnight. After adding 1 g of sodium chloride, the reaction solution was added to 200 mL of 90% ethanol/water for injection to form precipitates. After allowing it to stand for a while, the supernatant was removed. Then, 150 mL of 90% ethanol/water for injection was added, stirred and washed for 5 minutes, and allowed again to stand for a while. The same washing was additionally performed 2 times, and after filtering through a glass filter and drying the obtained precipitate overnight at 40°C under reduced pressure conditions (75 mmHg), the desired compound iv (1.02 g) was obtained. As a result of measuring $^1$H-NMR, the introduction ratio of betamethasone in the compound was found to be 17%.

(Example 5) Preparation of chondroitin sulfate introduced with 21-β-alanyl-budesonide

5-1. Preparation of 21-(Boc-β-alanyl)-budesonide

489 mg (1.0 eq., 2.58 mmol) of Boc-β-alanine was added with 18 mL of dichloromethane, g (0.9 eq., 2.32 mmol) of budesonide, and 157 mg (0.5 eq., 1.29 mmol) of N,N-dimethyl-4-aminopyridine. After that, under ice cooling, 1.24 g (2.5 eq., 6.45 mmol) of water soluble carbodiimide was added and stirred overnight at room temperature. After confirming by thin layer chromatography the disappearance of the reacting materials, a saturated aqueous solution of ammonium chloride was added under ice cooling and liquid fractionation extraction was performed 3 times by using dichloromethane and water. The collected organic layer was washed in order with a saturated aqueous solution of ammonium chloride, a saturated aqueous solution of sodium hydrogen carbonate, and a saturated aqueous brine solution. After drying over magnesium sulfate, it was concentrated under reduced pressure by using an evaporator in water bath at 40°C, and as a result, 1.31 g of the desired compound 6 was obtained (yield: 94%).

$^1$H-NMR(500MHz,CDCl$_3$)

δ0.92(3H,m),0.94(3H,m),1.15(2H,m),1.37(2H,m),1.43(3H,m),1.44(2H,m),1.45(1H,m),1 .46(9H,s,Boc),1.62(2H,m),1.75 (2H,m),2.08(1H,m),2.15(1H,ddd),2.33(1H,dd),2.58(1H, ddd),2.64(2H,m),3.41(1H,m),3.47(2H,m),4.50(1H,m),4.85(2H,m),5.15(1H,m),6.02(1H, s),6.28(1H,d),7.21(1H,d).

5-2. Preparation of 21-β-alanyl-budesonide

1.31 g of the compound 6 was dissolved in 20 mL of dichloromethane, and under ice cooling, added with 10 mL of 4 M hydrochloric acid/ethyl acetate, and stirred for 1 hour. After that, the temperature was raised to room temperature and it was stirred again for 1 hour. The reaction solution was concentrated under reduced pressure by using an evaporator in water bath at 40°C. As a result, the desired compound 7 was obtained in an amount of 1.12 g (yield of 97%).

$^1$H-NMR(500MHz,CDCl$_3$)

δ0.93(3H,m),0.94(3H,m),1.17(2H,m),1.40(2H,m),1.48(3H,s),1.62(2H,m),1.72(1H,m),1. δ5(2H,m),1.97(2H,m),2.10(1H,m),2.20(1H,ddd),2.36(1H,dd),2.58(1H,ddd),2.64(2H,m), 3.41(1H,m),3.47(2H,m),4.50(1H,m),4.85(2H,m),5.15(1H,m),6.02(1H,s),6.28(1H,d),7.2 1(1H,d).

5-3. Preparation of chondroitin sulfate introduced with 21-β-alanyl-budesonide (25 kDa)

2 g of sodium chondroitin sulfate (weight average molecular weight of 25 kDa) was dissolved in 30 mL of distilled water followed by addition of a mixture solution of 40 mL ethanol and 10 mL water containing 428 mg (0.2 eq., 0.80 mmol) of the compound 7. After that, 373 mg (0.2 eq., 0.80 mmol) of 4-(4,6-dimethoxy-1,3,5-triazin-2-yl)-4-methylmorpholinium chloride n-hydrate was added thereto and it was stirred overnight. After that, it was added with 2 g of sodium chloride followed by stirring until dissolution was confirmed. Then, the solution was added to 240 mL of 90% ethanol/distilled water for forming precipitates. The supernatant was discarded and washing with 90% ethanol/distilled water was performed 2 times. The obtained precipitates were dried overnight under reduced pressure, and as a polysaccharide derivative, 2 g of the desired compound va was obtained. As a result of measuring $^1$H-NMR, the introduction ratio was found to be about 19%.

5-4. Preparation of chondroitin sulfate introduced with 21-β-alanyl-budesonide (20 kDa)

To 1 g of sodium chondroitin sulfate (weight average molecular weight of 10 kDa), 15 mL of distilled water was added and stirred for 30 minutes. 15 mL of ethanol was slowly added and the solution was homogenously stirred. Thereafter, 200.26 mg of the compound 7 was dissolved in 5 mL solution of ethanol/distilled water = 1/1 and added thereto. Subsequently, 110.7 mg of 4-(4,6-dimethoxy-1,3,5-triazin-2-yl)-4-methylmorpholinium chloride n-hydrate was dissolved in 5 mL solution of ethanol/distilled water =1/1 and added thereto followed by stirring overnight. After adding 1 g of sodium chloride, the reaction solution was added to 200 mL of 90% ethanol/water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at 40°C under reduced pressure conditions (75 mmHg), the desired compound vb (0.87 g) was obtained as a polysaccharide derivative. As a result of measuring $^1$H-NMR, the introduction ratio of budesonide in the compound was found to be 17%.

5-5. Preparation of chondroitin sulfate introduced with 21-β-alanyl-budesonide (10 kDa)

To 1 g of sodium chondroitin sulfate (weight average molecular weight of 10 kDa), 15 mL of distilled water was added and stirred for 30 minutes. 15 mL of ethanol was slowly added and the solution was homogenously stirred. Thereafter, 200.26 mg of the compound 7 was dissolved in 5 mL solution of ethanol/distilled water =1/1 and added thereto. Subsequently, 110.7 mg of 4-(4,6-dimethoxy-1,3,5-triazin-2-yl)-4-methylmorpholinium chloride n-hydrate was dissolved in 5 mL solution of ethanol/distilled water =1/1 and added thereto followed by stirring overnight. After adding 1 g of sodium chloride, the reaction solution was added to 200 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at 40°C under reduced pressure conditions (75 mmHg), the desired compound vc (0.87 g) was obtained as a polysaccharide derivative. As a result of measuring $^1$H-NMR, the introduction ratio of budesonide in the compound was found to be 17%.

(Example 6) Preparation of hyaluronic acid introduced with 21-β-alanyl-budesonide

6-1. Preparation of hyaluronic acid introduced with 21-β-alanyl-budesonide (880 kDa)

1.5 g of sodium hyaluronate (weight average molecular weight of 880 kDa) was dissolved in 140 mL of distilled water and 140 mL of ethanol and added with a mixture solution of 10 mL ethanol and 10 mL water containing 402 mg of the compound 7. After that, 343 mg of 4-(4,6-dimethoxy-1,3,5-triazin-2-yl)-4-methylmorpholinium chloride n-hydrate was added thereto and stirred overnight. Then, 1.13 g of sodium hydrogen carbonate was added, stirred for 3 hours, and added in order with 600 μL of acetic acid, 4.5 g of sodium chloride, and 300 mL of 90% ethanol/distilled water to form precipitates. The supernatant was discarded and washing with 90% ethanol/distilled water was performed 2 times. The obtained precipitates were dried overnight under reduced pressure, and 1.4 g of the desired compound via was obtained. As a result of obtaining the introduction ratio by NMR, it was found to be about 19%.

6-2. Preparation of hyaluronic acid introduced with 21-β-alanyl-budesonide (390 kDa)

1 g of sodium hyaluronate (weight average molecular weight of 390 kDa) was added with 100 mL of distilled water followed stirring overnight. Then, it was slowly added with 100 mL of ethanol and the solution was homogeneously stirred followed by addition of 250.3 mg of the compound 7. After that, 138.4 mg of 4-(4,6-dimethoxy-1,3,5-triazin-2-yl)-4-methylmorpholinium chloride n-hydrate was added thereto and stirred overnight. Then, 750 mg of sodium hydrogen carbonate was added, stirred for 3 hours, and added with 0.2 mL of acetic acid for neutralization. After adding 3 g of sodium chloride, it was added with 230 mL of 90% ethanol/distilled water to form precipitates. After allowing it to stand for a while, the supernatant was discarded and 150 mL of 90% ethanol/distilled water was added, stirred and washed for 5 minutes, and allowed again to stand for a while. The same washing was additionally performed 2 times, and after filtering through a glass filter and drying the obtained precipitate overnight at 40°C under reduced pressure conditions (75 mmHg), the desired compound vib (1.01 g) was obtained as a polysaccharide derivative. As a result of measuring $^1$H-NMR, the introduction ratio of budesonide in the compound was found to be 16%.

(Example 7) Preparation of chondroitin sulfate introduced with 11-β-alanyl-propionic acid fluticasone

7-1. Preparation of 11-(Boc-β-alanyl)-propionic acid fluticasone

166 mg of Boc-β-alanine was added with 6 mL of dichloromethane, 338 mg of propionic acid fluticasone, and 83 mg of N,N-dimethyl-4-aminopyridine. After that, under ice cooling, 209 mg (1.5 eq., 1.01 mmol) of dicyclohexyl carbodiimide was added and stirred for 1 week at room temperature (white suspension). Then, the solution was quenched, and liquid fractionation extraction was performed 3 times after adding dichloromethane and water to the obtained 1-hydroxy benzotriazole solution. The collected organic layer was washed in order with a saturated aqueous solution of ammonium chloride, a saturated aqueous solution of sodium hydrogen carbonate, and a saturated aqueous brine solution. After drying over magnesium sulfate, it was concentrated under reduced pressure by using an evaporator in water bath at 40°C, and as a result, 450 mg of the compound 8 was obtained (yield: 99%).

7-2. Preparation of 11-β-alanyl-propionic acid fluticasone

450 mg of the compound 8 was dissolved in 8 mL of dichloromethane and 5 mL of tetrahydrofuran, and under ice cooling, added with 6 mL of 4 M hydrochloric acid/ethyl acetate, and stirred for 2 and a half hours. After that, the temperature was raised to room temperature and it was stirred again for 1 hour. The reaction solution was concentrated under reduced pressure by using an evaporator in water bath at 40°C. As a result, the desired compound 9 was obtained in an amount of 396 mg (yield of 97%).

7-3. Preparation of chondroitin sulfate introduced with 11-β-alanyl-propionic acid fluticasone

700 mg of sodium chondroitin sulfate (weight average molecular weight of 25 kDa) was dissolved in 15 mL of distilled water and added with a mixture solution of 25 mL ethanol and 10 mL water containing 169 mg (0.2 eq., 0.28 mmol) of the compound 9. After that, 131 mg (0.2 eq., 0.28 mmol) of 4-(4,6-dimethoxy-1,3,5-triazin-2-yl)-4-methylmorpholinium chloride n-hydrate was added thereto and stirred overnight. Then, 700 mg of sodium chloride was added. After confirming the dissolution, the solution was added to 150 mL of 90% ethanol/distilled water to form precipitates. The supernatant was discarded and washing with 90% ethanol/distilled water was performed 2 times. The obtained precipitates were dried overnight under reduced pressure, and as a polysaccharide derivative, 670 mg of the desired compound vii was obtained. As a result of obtaining the introduction ratio by $^1$H-NMR, it was found to be 19%.

(Example 8) Preparation of chondroitin sulfate introduced with 11-(2-fluoro-β-alanyl)-propionic acid fluticasone

8-1. Preparation of Boc-2-fluoro-β-alanine

1.34 g of 2-fluoro-β-alanine hydrochloride salt was dissolved in 30 mL of tert-butanol and 15 mL of 1 M sodium hydroxide, and under ice cooling, added with 2.24 g of di-tert-butyl bicarbonate. After that, it was stirred for 1 week at room temperature. After confirming by thin layer chromatography the disappearance of the reacting materials, it was concentrated under reduced pressure by using an evaporator in water bath at 40°C. Then, liquid fractionation extraction was performed 3 times using ethyl acetate and 1 M hydrochloric acid, and the collected organic layer was washed with a saturated aqueous brine solution. After drying over magnesium sulfate, it was concentrated under reduced pressure by using an evaporator in water bath at 40°C. As a result, the desired compound 10 was obtained in an amount of 1.91 g (yield of 99%). $^1$H-NMR(500MHz,CDCl$_3$)δ1.45(9H,s,Boc),3.64-3.79(2H,br),4.98(1H,br),5.05(1H,br.a mide).

8-2. Preparation of 11-(Boc-2-fluoro-β-alanyl)-propionic acid fluticasone

307 mg of the compound 10 was added with 6 mL of dichloromethane, 447 mg of propionic acid fluticasone, and 109 mg of N,N-dimethyl-4-aminopyridine. After that, under ice cooling, 369 mg of dicyclohexyl carbodiimide was added and stirred for 7 days at room temperature (white suspension). After confirming a new spot by thin layer chromatography, the solution was quenched, and liquid fractionation extraction was performed 3 times after adding dichloromethane and water to the obtained 1-hydroxy benzotriazole solution. The collected organic layer was washed in order with a saturated aqueous solution of ammonium chloride, a saturated aqueous solution of sodium hydrogen carbonate, and a saturated aqueous brine solution. After drying over magnesium sulfate, it was concentrated under reduced pressure by using an evaporator in water bath at 40°C. The reaction solution was purified by silica gel column chromatography (chloroform) to obtain the compound 11 in an amount of 384 mg (yield of 63%).

8-3. Preparation of 11-(2-fluoro-β-alanyl)-propionic acid fluticasone

384 mg of the compound 11 was dissolved in 10 mL of dichloromethane, and under ice cooling, added with 4.5 mL of 4 M hydrochloric acid/ethyl acetate, and stirred for 2 hours. After that, the temperature was raised to room temperature and it was stirred again for 1 hour. The reaction solution was concentrated under reduced pressure by using an evaporator in water bath at 40°C. As a result, the desired compound 12 was obtained in an amount of 284 mg (yield of 82%).

8-4. Preparation of chondroitin sulfate introduced with 11-(2-fluoro-β-alanyl)-propionic acid fluticasone

795 mg of sodium chondroitin sulfate (weight average molecular weight of 25 kDa) was dissolved in 10 mL of distilled water and added with a mixture solution of 15 mL ethanol and 5 mL water containing 277 mg of the compound 12. After that, 208 mg of 4-(4,6-dimethoxy-1,3,5-triazin-2-yl)-4-methylmorpholinium chloride n-hydrate was added thereto and stirred overnight. Then, 795 mg of sodium chloride was added. After confirming the dissolution, the solution was added to 90 mL of 90% ethanol/distilled water to form precipitates. The supernatant was discarded and washing with 90% ethanol/distilled water was performed 2 times. The obtained precipitates were dried overnight under reduced pressure, and as a polysaccharide derivative, 890 mg of the desired compound viii was obtained. As a result of obtaining the introduction ratio by $^1$H-NMR, it was found to be about 22%.

(Example 9) Preparation of chondroitin sulfate introduced with 21-alanyl-betamethasone

9-1. Preparation of 21-Boc-alanyl-betamethasone

0.964 g of Boc-alanine was dissolved in 10 mL of dichloromethane and 15 mL of dimethyl formamide followed by addition of 2 g of betamethasone. After cooling to 0°C, 186.8 mg of N,N-dimethyl-aminopyridine and 1.27 g of water soluble carbodiimide were added thereto and stirred overnight at room temperature. After confirming by thin layer chromatography the disappearance of the reacting materials, a saturated aqueous solution of ammonium chloride was added to terminate the reaction. Liquid fractionation extraction was performed by using toluene and water. The organic layer was washed with a saturated aqueous solution of ammonium chloride, a saturated aqueous solution of sodium hydrogen carbonate, and a saturated aqueous brine solution. After drying over magnesium sulfate anhydrous and filtering, the solvent was distilled off under reduced pressure, and as a result, the desired compound 13 was obtained (2.72 g, 98%).

9-2. Preparation of 21-alanyl-betamethasone

2.72 g of the compound 13 was dissolved in 15 mL of tetrahydrofuran. After cooling to 0°C, it was added with 100 mL of 4 M hydrochloric acid/ethyl acetate, and stirred for 3 hours at room temperature. After confirming by thin layer chromatography the disappearance of the reacting materials, the solvent was distilled off under reduced pressure to obtain the desired compound 14 (1.98 g, 86%).

9-3. Preparation of chondroitin sulfate introduced with 21-alanyl-betamethasone

1 g of sodium chondroitin sulfate (weight average molecular weight of 20 kDa) was added with 15 mL of distilled water followed by stirring for 30 minutes for dissolution. After that, 15 mL of ethanol was slowly added and the solution was homogeneously stirred. Then, the compound 14 (185.0 mg) was dissolved in 5 mL solution of ethanol/distilled water = 1/1 and added thereto. Subsequently, 110.7 mg of 4-(4,6-dimethoxy-1,3,5-triazin-2-yl)-4-methylmorpholinium chloride n-hydrate was dissolved in 5 mL solution of ethanol/distilled water =1/1 and added thereto followed by stirring overnight. After adding 1 g of sodium chloride, the reaction solution was added to 200 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at heating under reduced pressure conditions (40°C, 75 mmHg), the desired compound ix (0.90 g) was obtained as a polysaccharide derivative. As a result of measuring $^1$H-NMR, the introduction ratio was found to be 18%.

(Example 10) Preparation of chondroitin sulfate introduced with 21-glycyl-betamethasone

10-1. Preparation of 21-Boc-glycyl-betamethasone

1.34 g of Boc-glycine was dissolved in 20 mL of dichloromethane and 20 mL of dimethyl formamide followed by addition of 3 g of betamethasone. After cooling to 0°C, 280.2 mg of N,N-dimethyl-aminopyridine and 1.91 g of water soluble carbodiimide were added thereto and stirred overnight at room temperature. After confirming by thin layer chromatography the disappearance of the reacting materials, a saturated aqueous solution of ammonium chloride was added to terminate the reaction. Liquid fractionation extraction was performed by using toluene and water. The organic layer was washed with a saturated aqueous solution of ammonium chloride, a saturated aqueous solution of sodium hydrogen carbonate, and a saturated aqueous brine solution. After drying over magnesium sulfate anhydrous and filtering, the solvent was distilled off under reduced pressure, and as a result, the desired compound 15 was obtained (3.35 g, 82%).

10-2. Preparation of 21-glycyl-betamethasone

3.35 g of the compound 15 was dissolved in 20 mL of tetrahydrofuran. After cooling to 0°C, it was added with 100 mL of 4 M hydrochloric acid/ethyl acetate, and stirred for 1 hour at room temperature. After confirming by thin layer chromatography the disappearance of the reacting materials, the solvent was distilled off under reduced pressure to obtain the desired compound 16 (2.69 g, 95%).

10-3. Preparation of chondroitin sulfate introduced with 21-glycyl-betamethasone

1 g of sodium chondroitin sulfate (weight average molecular weight of 20 kDa) was added with 15 mL of distilled water followed by stirring for 30 minutes for dissolution. 15 mL of ethanol was slowly added and the solution was homogeneously stirred. Then, the compound 16 (224.3 mg) was dissolved in 5 mL solution of ethanol/distilled water =1/1 and added thereto. Subsequently, 110.7 mg of 4-(4,6-dimethoxy-1,3,5-triazin-2-yl)-4-methylmorpholinium chloride n-hydrate was dissolved in 5 mL solution of ethanol/distilled water =1/1 and added thereto followed by stirring overnight. After adding 1 g of sodium chloride, the reaction solution was added to 200 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at heating under reduced pressure conditions (40°C, 75 mmHg), the desired compound x (0.61 g) was obtained as a polysaccharide derivative. As a result of measuring $^1$H-NMR, the introduction ratio was found to be 20%.

(Example 11) Preparation of chondroitin sulfate introduced with 21-isoleucyl-betamethasone

11-1. Preparation of 21-(Boc-isoleucyl)-betamethasone

1.77 g of Boc-isoleucine was dissolved in 20 mL of dichloromethane and 20 mL of dimethyl formamide followed by addition of 3 g of betamethasone. After cooling to 0°C, 280.2 mg of N,N-dimethylaminopyridine and 1.91 g of water soluble carbodiimide were added followed by stirring overnight at room temperature. After confirming by thin layer chromatography the disappearance of the reacting materials, a saturated aqueous solution of ammonium chloride was added to terminate the reaction. Liquid fractionation extraction was performed by using toluene and water, and the organic layer was washed with a saturated aqueous solution of ammonium chloride, a saturated aqueous solution of sodium hydrogen carbonate, and saturated brine. After drying over magnesium sulfate anhydrous and filtering, the solvent was distilled off under reduced pressure to obtain the desired compound 17 (3.96 g, 87%).

11-2. Preparation of 21-isoleucyl-betamethasone

3.96 g of the compound 17 was dissolved in 20 mL of tetrahydrofuran. After cooling to 0°C, 100 mL of 4 M hydrochloric acid/ethyl acetate was added and it was stirred for 1 hour at room temperature. After confirming by thin layer chromatography the disappearance of the reacting materials, the solvent was distilled off under reduced pressure to obtain the desired compound 18 (3.36 g, 99%).

11-3. Preparation of chondroitin sulfate introduced with 21-isoleucyl-betamethasone

To 1 g of sodium chondroitin sulfate (weight average molecular weight of 20 kDa), 15 mL of distilled water was added and stirred for 30 minutes for dissolution. 15 mL of ethanol was slowly added and the solution was homogenously stirred. Thereafter, 201.9 mg of the compound 18 was dissolved in 5 mL solution of ethanol/distilled water = 1/1 and added thereto. Subsequently, 110.7 mg of 4-(4,6-dimethoxy-1,3,5-triazin-2-yl)-4-methylmorpholinium chloride n-hydrate was dissolved in 5 mL of ethanol/distilled water = 1/1 solution and added thereto followed by stirring overnight. After adding 1 g of sodium chloride, the reaction solution was added to 200 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at heating under reduced pressure conditions (40°C, 75 mmHg), the desired compound xi (0.76 g) was obtained as a polysaccharide derivative. As a result of measuring $^1$H-NMR, the introduction ratio was found to be 15%.

(Example 12) Preparation of chondroitin sulfate introduced with 21-phenylalanyl-betamethasone

12-1. Preparation of 21-(Boc-phenylalanyl)-betamethasone

2.03 g of Boc-phenylalanine was dissolved in 20 mL of dichloromethane and 20 mL of dimethyl formamide followed by addition of 3 g of betamethasone. After cooling to 0°C, 280.2 mg of N,N-dimethylaminopyridine and 1.91 g of water soluble carbodiimide were added followed by stirring overnight at room temperature. After confirming by thin layer chromatography the disappearance of the reacting materials, a saturated aqueous solution of ammonium chloride was added to terminate the reaction. Liquid fractionation extraction was performed by using toluene and water, and the organic layer was washed with a saturated aqueous solution of ammonium chloride, a saturated aqueous solution of sodium hydrogen carbonate, and saturated brine. After drying over magnesium sulfate anhydrous and filtering, the solvent was distilled off under reduced pressure to obtain the desired compound 19 (0.837 g, 18%).

12-2. Preparation of 21-phenylalanyl-betamethasone

0.837 g of the compound 19 was dissolved in 15 mL of tetrahydrofuran. After cooling to 0°C, 25 mL of 4 M hydrochloric acid/ethyl acetate was added and it was stirred for 3 hours at room temperature. After confirming by thin layer chromatography the disappearance of the reacting materials, the solvent was distilled off under reduced pressure to obtain the desired compound 20 (0.63 g, 87%).

12-3. Preparation of chondroitin sulfate introduced with 21-phenylalanyl-betamethasone

To 1 g of sodium chondroitin sulfate (weight average molecular weight of 20 kDa), 15 mL of distilled water was added and stirred for 30 minutes for dissolution. 15 mL of ethanol was slowly added and the solution was homogenously stirred. Thereafter, 215.5 mg of the compound 20 was dissolved in 5 mL solution of ethanol/distilled water = 1/1 and added thereto. Subsequently, 110.7 mg of 4-(4,6-dimethoxy-1,3,5-triazin-2-yl)-4-methylmorpholinium chloride n-hydrate was dissolved in 5 mL of ethanol/distilled water =1/1 solution and added thereto followed by stirring overnight. After adding 1 g of sodium chloride, the reaction solution was added to 200 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at heating under reduced pressure conditions (40°C, 75 mmHg), the desired compound xii (0.75 g) was obtained as a polysaccharide derivative. As a result of measuring $^1$H-NMR, the introduction ratio was found to be 15%.

(Example 13) Preparation of chondroitin sulfate introduced with 21-($\alpha$-N-acetyl)-ornithyl-betamethasone

13-1. Preparation of 21-($\alpha$-Fmoc-$\delta$-Boc)-ornithyl-betamethasone

1 g of $\alpha$-Fmoc-$\gamma$-Boc-ornithine was added with 12 mL of dichloromethane, 6 mL of dimethyl formamide, 747 mg of betamethasone, and 74 mg of N,N-dimethyl-4-aminopyridine, After that, under ice cooling, 1.16 g of water soluble carbodiimide was added followed by stirring overnight at room temperature. After confirming by thin layer chromatography the disappearance of the reacting materials, under ice cooling, a saturated aqueous solution of ammonium chloride was added. Liquid fractionation extraction was performed 3 times by using toluene and water, and the collected organic layer was washed in order with a saturated aqueous solution of ammonium chloride, a saturated aqueous solution of sodium hydrogen carbonate, and a saturated aqueous brine solution. After drying over magnesium sulfate, it was concentrated under reduced pressure by using an evaporator in water bath at 60°C, and as a result, 1.57 g of the desired compound 21 was obtained (yield: 99%).

13-2. Preparation of 21-($\delta$-Boc)-ornithyl-betamethasone

1.26 g of the compound 21 was added with 9 mL of dichloromethane and 1.6 mL of diethylamine followed by stirring for 6 and a half hours at room temperature. After confirming a new spot by thin layer chromatography, water was added thereto under ice cooling, and liquid fractionation extraction was performed 3 times by using dichloromethane and water. The collected organic layer was washed with saturated aqueous brine solution. After drying over magnesium sulfate, it was concentrated under reduced pressure by using an evaporator in water bath at 40°C, and as a result, 654 mg of the desired compound 22 was obtained (yield: 71 %).

13-3. Preparation of 21-($\alpha$-N-acetyl-$\delta$-Boc)-ornithyl-betamethasone

195 mg of the compound 22 was dissolved in 5 mL of dichloromethane, added with 112 $\mu$L of diisopropylethyl amine, and reacted with 30 $\mu$L of acetic anhydride under ice cooling. After stirring overnight at room temperature and confirming by thin layer chromatography the disappearance of the reacting materials, under ice cooling, a saturated aqueous solution of ammonium chloride was added. Liquid fractionation extraction was performed 3 times by using dichloromethane and water, and the collected organic layer was washed in order with a saturated aqueous solution of ammonium chloride, a saturated aqueous solution of sodium hydrogen carbonate, and a saturated aqueous brine solution. After drying over magnesium sulfate, it was concentrated under reduced pressure by using an evaporator in water bath at 40°C, and as a result, 202 mg of the desired compound 23 was obtained (yield: 97%).

13-4. Preparation of 21-($\alpha$-N-acetyl)-ornithyl-betamethasone

220 mg of the compound 23 was dissolved in 20 mL of dichloromethane. Under ice cooling, 8 mL of 4 M hydrochloric acid/ethyl acetate was added followed by stirring for 1 hour. After raising the temperature to room temperature, it was stirred again for 1 and a half hours. The reaction solution was concentrated under reduced pressure by using an evaporator in water bath at 40°C followed by washing with diethyl ether, and as a result, 198 mg of the desired compound 24 was obtained (yield: 97%).

13-5. Preparation of chondroitin sulfate introduced with 21-($\alpha$-N-acetyl)-ornithyl-betamethasone

380 mg of sodium chondroitin sulfate (weight average molecular weight of 25 kDa) was dissolved in 5 mL of distilled water and added with a mixture solution of 7 mL ethanol and 2 mL water containing 177 mg of the compound 24. After that, 142 mg of 4-(4,6-dimethoxy-1,3,5-triazin-2-yl)-4-methylmorpholinium chloride n-hydrate was added thereto and stirred overnight. Then, 380 mg of sodium chloride was added. The solution was added to 42 mL of ethanol to form precipitates. The supernatant was discarded and washing with 90% ethanol/distilled water was performed 2 times. Washing with ethanol was performed 2 times. The obtained precipitates were dried overnight under reduced pressure, and as a polysaccharide derivative, 370 mg of the target product xiii was obtained. As a result of obtaining the introduction ratio by $^1$H-NMR, it was found to be about 19%.

(Example 14) Preparation of chondroitin sulfate introduced with 21-β-alanyl-prednisolone

14-1. Preparation of 21-(Boc-β-alanyl)-prednisolone

300 mg (1.59 mmol) of Boc-β-alanine was added with 6 mL of dichloromethane, 3 mL of dimethyl formamide, 571 mg of prednisolone, and 58 mg of N,N-dimethyl-4-aminopyridine. After that, under ice cooling, 334 mg of water soluble carbodiimide was added followed by stirring overnight at room temperature. After confirming by thin layer chromatography the disappearance of the reacting materials, liquid fractionation extraction was performed 3 times by using toluene and water, and the collected organic layer was washed in order with a saturated aqueous solution of ammonium chloride, a saturated aqueous solution of sodium hydrogen carbonate, and a saturated aqueous brine solution. After drying over magnesium sulfate, it was concentrated under reduced pressure by using an evaporator in water bath at 60°C, and as a result, 837 mg of the desired compound was obtained (yield: 101%).

14-2. Preparation of 21-β-alanyl-prednisolone

400 mg of the compound a was dissolved in 5 mL of dichloromethane. Under ice cooling, 2 mL of 4 M hydrochloric acid/ethyl acetate was added and it was stirred for 3 hours. The reaction solution was concentrated under reduced pressure by using an evaporator in water bath at 40°C, and as a result, 208 mg of the desired compound was obtained.

14-3. Preparation of chondroitin sulfate introduced with 21-β-alanyl-prednisolone

5 g of sodium chondroitin sulfate (weight average molecular weight of 25 kDa) was dissolved in 200 mL of distilled water and 200 mL of ethanol, and added with 930 mg of the compound b, and 932 mg of 4-(4,6-dimethoxy-1,3,5-triazin-2-yl)-4-methylmorpholinium chloride n-hydrate followed by stirring overnight. Then, 1.5 g of sodium hydrogen carbonate was added followed by stirring for 3 hours. Then, 1.06 mL of acetic acid, 6 g of sodium chloride, and 400 mL of 90% ethanol/distilled water were added in order for forming precipitates. The supernatant was discarded and washing with 90% ethanol/distilled water was performed 2 times. The obtained precipitates were dried overnight under reduced pressure, and as a polysaccharide derivative, 4.95 g of the desired compound xiv was obtained. As a result of obtaining the introduction ratio by $^1$H-NMR, it was found to be about 18%.

(Example 15) Preparation of chondroitin sulfate introduced with 21-glycyl-budesonide

15-1. Preparation of 21-(Boc-glycyl)-budesonide

402.9 mg of Boc-glycine was dissolved in 10 mL of dichloromethane, and added with 1 g of budesonide. After cooling to 0°C, 84.3 mg of N,N-dimethylaminopyridine and 573.2 mg of water soluble carbodiimide were added thereto followed by stirring overnight at room temperature. After confirming by thin layer chromatography the disappearance of the reacting materials, the reaction was terminated by adding a saturated aqueous solution of ammonium chloride. The organic layer was washed with a saturated aqueous solution of ammonium chloride, a saturated aqueous solution of sodium hydrogen carbonate, and saturated brine. After drying over magnesium sulfate anhydrous and filtering, the solvent was distilled off under reduced pressure, and as a result, the desired compound 27 was obtained (1.11 g, 84%).

15-2. Preparation of 21-glycyl-budesonide

1.11 g of the compound 27 was dissolved in 20 mL of tetrahydrofuran. After cooling to 0°C, 100 mL of 4 M hydrochloric acid/ethyl acetate was added and it was stirred for 1 hour at room temperature. After confirming by thin layer chromatography the disappearance of the reacting materials, the solvent was distilled off under reduced pressure, and as a result, the desired compound 28 was obtained (1.00 g, 99%).

15-3. Preparation of chondroitin sulfate introduced with 21-glycyl-budesonide

To 1 g of sodium chondroitin sulfate (weight average molecular weight of 20 kDa), 15 mL of distilled water was added and stirred for 30 minutes for dissolution. 15 mL of ethanol was slowly added and the solution was homogenously stirred. Thereafter, 209.6 mg of the compound 28 was dissolved in 5 mL solution of ethanol/distilled water = 1/1 and added thereto. Subsequently, 110.7 mg of 4-(4,6-dimethoxy-1,3,5-triazin-2-yl)-4-methylmorpholinium chloride n-hydrate was dissolved in 5 mL of ethanol/distilled water =1/1 solution and added thereto followed by stirring overnight. After adding 1 g of sodium chloride, the reaction solution was added to 200 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at heating under reduced pressure conditions (40°C, 75 mmHg), the desired compound xv (0.992 g) was obtained as a polysaccharide derivative. As a result of measuring $^1$H-NMR, the introduction ratio was found to be 18%.

(Example 16) Preparation of chondroitin sulfate introduced with 21-(isoleucyl)-budesonide

16-1. Preparation of 21-(Boc-isoleucyl)-budesonide

532.0 mg of Boc-isoleucine was dissolved in 10 mL of dichloromethane followed by addition of 1 g of budesonide. After cooling to 0°C, 84.3 mg of N,N-dimethylaminopyridine and 573.2 mg of water soluble carbodiimide were added followed by stirring overnight at room temperature. After confirming by thin layer chromatography the disappearance of the reacting materials, a saturated aqueous solution of ammonium chloride was added to terminate the reaction. The organic layer was washed with a saturated aqueous solution of ammonium chloride, a saturated aqueous solution of sodium hydrogen carbonate, and saturated brine. After drying over magnesium sulfate anhydrous and filtering, the solvent was distilled off under reduced pressure to obtain the desired compound 29 (1.38 g, 95%).

16-2. Preparation of 21-isoleucyl-budesonide

1.38 g of the compound 29 was dissolved in 20 mL of tetrahydrofuran. After cooling to 0°C, 100 mL of 4 M hydrochloric acid/ethyl acetate was added and it was stirred for 1 hour at room temperature. After confirming by thin layer chromatography the disappearance of the reacting materials, the solvent was distilled off under reduced pressure to obtain the desired compound 30 (1.17 g, 92%).

16-3. Preparation of chondroitin sulfate introduced with 21-isoleucyl-budesonide

To 1 g of sodium chondroitin sulfate (weight average molecular weight of 20 kDa), 15 mL of distilled water was added and stirred for 30 minutes for dissolution. 15 mL of ethanol was slowly added and the solution was homogenously stirred. Thereafter, 232.1 mg of the compound 30 was dissolved in 5 mL solution of ethanol/distilled water = 1/1 and added thereto. Subsequently, 110.7 mg of 4-(4,6-dimethoxy-1,3,5-triazin-2-yl)-4-methylmorpholinium chloride n-hydrate was dissolved in 5 mL of ethanol/distilled water =1/1 solution and added thereto followed by stirring overnight. After adding 1 g of sodium chloride, the reaction solution was added to 200 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at heating under reduced pressure conditions (40°C, 75 mmHg), the desired compound xvi (0.989 g) was obtained as a polysaccharide derivative. As a result of measuring $^1$H-NMR, the introduction ratio was found to be 13%.

(Example 17) Preparation of chondroitin sulfate introduced with 11-glycyl-propionic acid fluticasone

17-1. Preparation of 11-(Boc-glycyl)-propionic acid fluticasone

210.2 mg of Boc-glycine and 600 mg of propionic acid fluticasone were dissolved in 100 mL of dichloromethane and 20 mL of dimethyl formamide. After cooling to 0°C, 77.0 mg of N,N-dimethylaminopyridine and 321.9 mg of dicyclohexyl carbodiimide were added followed by stirring overnight at room temperature. After cooling to 0°C, Boc-glycine (210.2 mg), N,N-dimethylaminopyridine (77.0 mg), and dicyclohexylcarbodiimide (321.9 mg) were added thereto followed by stirring again for 4 days. After that, a saturated aqueous solution of ammonium chloride was added to terminate the reaction. Liquid fractionation extraction was performed by using toluene and water. The organic layer was washed with a saturated aqueous solution of ammonium chloride, a saturated aqueous solution of sodium hydrogen carbonate, and saturated brine. After drying over magnesium sulfate anhydrous and filtering, the solvent was distilled off under reduced pressure. The obtained residues were separated and purified by silica gel column chromatography (hexane/ethyl acetate = 10/1→5/1→3/1→2/1) to obtain the desired compound 31 (0.493 g, 63%).

17-2. Preparation of 11-glycyl-propionic acid fluticasone

0.384 g of the compound 31 was dissolved in 5 mL of tetrahydrofuran. After cooling to 0°C, 15 mL of 4 M hydrochloric acid/ethyl acetate was added and it was stirred for 4 hours at room temperature. After confirming by thin layer chromatography the disappearance of the reacting materials, the solvent was distilled off under reduced pressure to obtain the desired compound 32 (0.301 g, 72%).

17-3. Preparation of chondroitin sulfate introduced with 11-glycyl-propionic acid fluticasone

To 500 mg of sodium chondroitin sulfate (weight average molecular weight of 20 kDa), 7.5 mL of distilled water was added and stirred for 30 minutes for dissolution. 7.5 mL of ethanol was slowly added and the solution was homogenously stirred. Thereafter, 118.8 mg of the compound 32 was dissolved in 5 mL solution of ethanol/distilled water =1/1 and added thereto. Subsequently, 55.3 mg of 4-(4,6-dimethoxy-1,3,5-triazin-2-yl)-4-methylmorpholinium chloride n-hydrate was dissolved in 5 mL of ethanol/distilled water =1/1 solution and added thereto followed by stirring overnight. After adding 500 mg of sodium chloride, the reaction solution was added to 100 mL of 90% ethanol/distilled water to form precipitates. After allowing it to stand for a while, the supernatant was removed. Then, 7.5 mL of 90% ethanol/distilled water was added, stirred and washed for 5 minutes, and allowed again to stand for a while. The same washing was additionally performed 2 times, and after filtering through a glass filter and drying the obtained precipitate overnight at heating under reduced pressure conditions (40°C, 75 mmHg), the desired compound xvii (0.362 g) was obtained as a polysaccharide derivative. As a result of measuring $^1$H-NMR, the introduction ratio was found to be 18%.

(Example 18) Preparation of budesonide 12-aminododecylate

18-1. Preparation of Boc-12-amino-dodecanoic acid

3 g of 12-aminododecanoic acid was dissolved in 100 mL of 0.1 M sodium hydroxide solution and 100 mL of dichloromethane. After cooling to 0°C, di-tert-butyl bicarbonate dissolved in 10 mL of dichloromethane was added dropwise. After stirring overnight, the solvent was distilled off under reduced pressure, and it was dissolved in ethyl acetate and washed with 0.1 M hydrochloric acid and saturated brine. After drying over magnesium sulfate anhydrous, it was filtered and the solvent was distilled off under reduced pressure to obtain the desired compound 32 (2.01 g, 45%).

18-2. Preparation of budesonide Boc-12-aminododecylate

659 mg of the compound 32 was dissolved in 10 mL of dichloromethane, and added with 900 mg of budesonide. After cooling to 0°C, 77.0 mg of N,N-dimethyl-aminopyridine and 521 mg of water soluble carbodiimide were added followed by stirring overnight at room temperature. After confirming by thin layer chromatography the disappearance of the reacting materials, the reaction was terminated by adding a saturated aqueous solution of ammonium chloride. Then, the organic layer was washed with a saturated aqueous solution of ammonium chloride, a saturated aqueous solution of sodium hydrogen carbonate, and saturated brine. After drying over magnesium sulfate anhydrous, it was filtered and the solvent was distilled off under reduced pressure to obtain the desired compound xviiia (1.45 g, 95%).

18-3. Preparation of budesonide 12-aminododecylate

0.45 g of the compound xviiia was dissolved in 20 mL of dichloromethane. After cooling to 0°C, 20 mL of 4 M hydrochloric acid/ethyl acetate was added and it was stirred for 4 hours at room temperature. After confirming by thin layer chromatography the disappearance of the reacting materials, the solvent was distilled off under reduced pressure to obtain the desired compound xviiib (0.38 g, 94%).

(Example 19) Preparation of carboxymethyl cellulose introduced with 21-β-alanyl-betamethasone

19-1. Preparation of carboxymethyl cellulose (500) introduced with 21-β-alanyl-betamethasone

To 1 g of carboxymethyl cellulose (n = 500), 100 mL of distilled water was added and stirred for 1 hour. 100 mL of ethanol was slowly added and the solution was homogenously stirred. Thereafter, 231.3 mg of the compound 2 was added thereto. Subsequently, 138.4 mg of 4-(4,6-dimethoxy-1,3,5-triazin-2-yl)-4-methylmorpholinium chloride n-hydrate was added thereto followed by stirring overnight. Then, 750 mg of sodium hydrogen carbonate was added followed by stirring for 3 hours and neutralization was performed according to addition of 0.2 mL of acetic acid. After adding 3 g of sodium chloride, the reaction solution was added with 230 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at heating under reduced pressure conditions (40°C, 75 mmHg), the desired compound xixa (0.963 g) was obtained as a polysaccharide derivative. As a result of measuring the introduction ratio of betamethasone by using a spectrophotometer, it was found to be 17%.

19-2. Preparation of carboxymethyl cellulose (1050) introduced with 21-β-alanyl-betamethasone

To 1 g of carboxymethyl cellulose (n = 1050), 100 mL of distilled water was added and stirred for 1 hour. 100 mL of ethanol was slowly added and the solution was homogenously stirred. Thereafter, 231.3 mg of the compound 2 was added thereto. Subsequently, 138.4 mg of 4-(4,6-dimethoxy-1,3,5-triazin-2-yl)-4-methylmorpholinium chloride n-hydrate was added thereto followed by stirring overnight. Then, 750 mg of sodium hydrogen carbonate was added followed by stirring for 3 hours and neutralization was performed according to addition of 0.2 mL of acetic acid. After adding 3 g of sodium chloride, the reaction solution was added with 230 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at heating under reduced pressure conditions (40°C, 75 mmHg), the desired compound xixb (0.963 g) was obtained as a polysaccharide derivative. As a result of measuring the introduction ratio of betamethasone by using a spectrophotometer, it was found to be 18%.

(Example 20) Preparation of carboxymethyl cellulose introduced with 21-β-alanyl-budesonide

20-1. Preparation of carboxymethyl cellulose (500) introduced with 21-β-alanyl-budesonide

To 1 g of carboxymethyl cellulose (n = 500), 100 mL of distilled water was added and stirred for 1 hour. 100 mL of ethanol was slowly added and the solution was homogenously stirred. Thereafter, 250.3 mg of the compound 7 was added thereto. Subsequently, 138.4 mg of 4-(4,6-dimethoxy-1,3,5-triazin-2-yl)-4-methylmorpholinium chloride n-hydrate was added thereto followed by stirring overnight. Then, 750 mg of sodium hydrogen carbonate was added followed by stirring for 3 hours and neutralization was performed according to addition of 0.2 mL of acetic acid. After adding 3 g of sodium chloride, the reaction solution was added with 230 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at 40°C under reduced pressure conditions (75 mmHg), the desired compound xxa (0.978 g) was obtained as a polysaccharide derivative. As a result of measuring the introduction ratio of budesonide by using a spectrophotometer, it was found to be 15%.

20-2. Preparation of carboxymethyl cellulose (1050) introduced with 21-β-alanyl-budesonide

To 1 g of carboxymethyl cellulose (n = 1050), 100 mL of distilled water was added and stirred for 1 hour. 100 mL of ethanol was slowly added and the solution was homogenously stirred. Thereafter, 231.3 mg of the compound 7 was added thereto. Subsequently, 138.4 mg of 4-(4,6-dimethoxy-1,3,5-triazin-2-yl)-4-methylmorpholinium chloride n-hydrate was added thereto followed by stirring overnight. Then, 750 mg of sodium hydrogen carbonate was added followed by stirring for 3 hours and neutralization was performed according to addition of 0.2 mL of acetic acid. After adding 3 g of sodium chloride, the reaction solution was added with 230 mL of 90% ethanol/distilled water to form precipitates. After allowing it to stand for a while, the supernatant was removed. Then, 150 mL of 90% ethanol/distilled water was added, stirred and washed for 5 minutes, and allowed again to stand for a while. The same washing was additionally performed 2 times, and after filtering through a glass filter and drying the obtained precipitate overnight at 40°C under reduced pressure conditions (75 mmHg), the desired compound xxb (0.952 g) was obtained as a polysaccharide derivative. As a result of measuring the introduction ratio of budesonide by using a spectrophotometer, it was found to be 18%.

(Example 21) Preparation of chondroitin sulfate introduced with betamethasone

To 1 g of sodium chondroitin sulfate (weight average molecular weight of 20 kDa), 15 mL of distilled water was added and stirred for 30 minutes. 15 mL of 1,4-dioxane was slowly added and the solution was homogenously stirred.

Then, betamethasone (784.9 mg, 1eq.) was dissolved in 15 mL of 1,4-dioxane and added thereto. Subsequently, 553.4 mg of 4-(4,6-dimethoxy-1,3,5-triazin-2-yl)-4-methylmorpholinium chloride n-hydrate was added thereto followed by stirring overnight. After adding 1 g of sodium chloride, the reaction solution was added to 200 mL of 90% ethanol/water for injection to form precipitates. After allowing it to stand for a while, the supernatant was removed. Then, 150 mL of 90% ethanol/water for injection was added, stirred and washed for 5 minutes, and allowed again to stand for a while. The same washing was additionally performed 2 times, and after filtering through a glass filter and drying the obtained precipitate overnight at 40°C under reduced pressure conditions (75 mmHg), the desired compound xxi (0.92 g) was obtained as a polysaccharide derivative. As a result of measuring the introduction ratio of budesonide by $^1$H-NMR, it was found to be 2%.

(Example 22) Preparation of chondroitin sulfate introduced with proxyphylline

22-1. Preparation of Boc-β-alanyl-proxyphylline

398 mg of Boc-β-alanine was added with 17 mL of dichloromethane, 500 mg of proxyphylline, and 77 mg of N,N-dimethyl-4-aminopyridine. After that, under ice cooling, 1.2 g of water soluble carbodiimide was added and stirred overnight at room temperature. After confirming a new spot by thin layer chromatography, a saturated aqueous solution of ammonium chloride was added under ice cooling. Then, liquid fractionation extraction was performed 3 times by using dichloromethane and water. The collected organic layer was washed in order with a saturated aqueous solution of ammonium chloride, a saturated aqueous solution of sodium hydrogen carbonate, and a saturated aqueous brine solution. After drying over magnesium sulfate, it was concentrated under reduced pressure by using an evaporator in water bath at 40°C, and as a result, 790 mg of the desired compound 33 was obtained (yield: 92%).

22-2. Preparation of β-alanyl-proxyphylline

754 mg of the compound 33 was dissolved in 20 mL of dichloromethane. Under ice cooling, 8 mL of 4 M hydrochloric acid/ethyl acetate was added and it was stirred for 1 hour. The temperature was raised to room temperature, and the reaction solution was stirred again for 1 hour. The reaction solution was concentrated under reduced pressure by using an evaporator in water bath at 40°C followed by washing with diethyl ether, and as a result, 635 mg of the desired compound 34 was obtained.

22-3. Preparation of chondroitin sulfate introduced with β-alanyl-proxyphylline

1 g of sodium chondroitin sulfate (weight average molecular weight of 25 kDa) was dissolved in 15 mL of distilled water followed by addition of a mixture solution of 20 mL of ethanol and 5 mL of water containing 138 mg of the compound 34. After that, 187 mg of 4-(4,6-dimethoxy-1,3,5-triazin-2-yl)-4-methylmorpholinium chloride n-hydrate was added thereto and it was stirred overnight. After that, it was added with 1 g of sodium chloride followed by dropwise addition of the solution to 120 mL of ethanol for forming precipitates. The supernatant was discarded and washing with 90% ethanol/distilled water was performed 2 times. The obtained precipitates were dried overnight under reduced pressure, and as a polysaccharide derivative, 971 mg of the desired compound xxiia was obtained. As a result of obtaining the introduction ratio by $^1$H-NMR, it was found to be about 16%.

22-4. Preparation of hyaluronic acid introduced with proxyphylline

600 mg of sodium hyaluronate (weight average molecular weight of 880 kDa) was dissolved in 60 mL of distilled water and 50 mL of ethanol and added with an ethanol solution of 10 mL containing 104 mg of the compound 34. After that, 141 mg of 4-(4,6-dimethoxy-1,3,5-triazin-2-yl)-4-methylmorpholinium chloride n-hydrate was added thereto and stirred overnight. Then, 450 mg of sodium hydrogen carbonate was added, stirred for 3 hours, and added in order with 240 μL of acetic acid, 1.8 g of sodium chloride, and 240 mL of 90% ethanol/distilled water to form precipitates. The supernatant was discarded and washing with 90% ethanol/distilled water was performed 2 times. The obtained precipitates were dried overnight under reduced pressure, and as a polysaccharide derivative, 568 mg of the desired compound xxiib was obtained. As a result of obtaining the introduction ratio by $^1$H-NMR, it was found to be about 17%.

(Example 23) Preparation of chondroitin sulfate introduced with seratrodast

23-1. Preparation of seratrodast Boc-aminoethyl ester

44 mg of Boc-2-aminoethanol was added with 5 mL of dichloromethane, 99 mg of seratrodast, and 10 mg of N,N-dimethyl-4-aminopyridine. After that, under ice cooling, 157 mg of water soluble carbodiimide was added thereto followed by stirring overnight at room temperature. After confirming by thin layer chromatography the disappearance of the reacting materials, under ice cooling, a saturated aqueous solution of ammonium chloride was added. Liquid fractionation extraction was performed 3 times by using dichloromethane and water. The collected organic layer was washed in order with a saturated aqueous solution of ammonium chloride, a saturated aqueous solution of sodium hydrogen carbonate, and a saturated aqueous brine solution. After drying over magnesium sulfate, it was concentrated under reduced pressure by using an evaporator in water bath at 40°C, and as a result, 136 mg of the desired compound 35 was obtained (yield: quant.).

23-2. Preparation of seratrodast aminoethyl ester

136 mg of the compound 35 was dissolved in 5 mL of dichloromethane. Under ice cooling, 2 mL of 4 M hydrochloric acid/ethyl acetate was added and it was stirred for 1 hour. After that, the temperature was raised to room temperature, and it was stirred again for 1 hour. The reaction solution was concentrated under reduced pressure by using an evaporator in water bath at 40°C followed by washing with diethyl ether. As a result, 118 mg of the desired compound 36 was obtained (yield: 98%).

23-3. Preparation of chondroitin sulfate introduced with seratrodast

423 mg of sodium chondroitin sulfate (weight average molecular weight of 25 kDa) was dissolved in 7 mL of distilled water, and added with a mixture solution of ethanol (9 mL) and water (2 mL) containing 73 mg of the compound 36. After that, 79 mg of 4-(4,6-dimethoxy-1,3,5-triazin-2-yl)-4-methylmorpholinium chloride n-hydrate was added thereto and stirred overnight. Then, 423 mg of sodium chloride was added, and the solution was added dropwise to 55 mL of ethanol to form precipitates. The supernatant was discarded and washing with 90% ethanol/distilled water was performed 2 times. The obtained precipitates were dried overnight under reduced pressure, and as a polysaccharide derivative, 403 mg of the desired compound xxiii was obtained. As a result of obtaining the introduction ratio by $^1$H-NMR, it was found to be about 17%.

(Example 24) Preparation of chondroitin sulfate introduced with montelukast

24-1. Preparation of Fmoc-4-amino-1-butanol

264 mg of 4-amino-1-butanol was dissolved in 9 mL of tetrahydrofuran and 3 mL of water. Under ice cooling, 1.0 g of 9-fluorenyl methyl N-succinimidyl carbonate was added. After that, it was stirred overnight at room temperature. After confirming the disappearance of the reacting materials, liquid fractionation extraction was performed 3 times by using dichloromethane and water. The collected organic layer was washed with a saturated aqueous brine solution. After drying over magnesium sulfate, it was concentrated under reduced pressure, and as a result, 1.21 g of the desired compound 37 was obtained.

24-2. Preparation of montelukast Fmoc-4-amino-1-butyl ester

196 mg of the compound 37 was dissolved in 10 mL of dichloromethane, and added with 400 mg of sodium montelukast and 24 mg of N,N-dimethyl-4-aminopyridine. After that, under ice cooling, 189 mg of water soluble carbodiimide was added followed by stirring overnight at room temperature. Under ice cooling, a saturated aqueous solution of ammonium chloride was added, and liquid fractionation extraction was performed 3 times by using dichloromethane and water. The collected organic layer was washed with a saturated aqueous solution of ammonium chloride, a saturated aqueous solution of sodium hydrogen carbonate, and a saturated aqueous brine solution. After drying over magnesium sulfate, it was concentrated under reduced pressure. The resultant was purified by silica gel column chromatography (hexane : ethyl acetate = 2 :1) to obtain the desired compound 38 in an amount of 406 mg.

24-3. Preparation of montelukast 4-amino-1-butyl ester

200 mg of the compound 38 was dissolved in 8 mL of dichloromethane followed by addition of 2 mL of piperidine. It was stirred for 3 hours at room temperature, and after confirming the disappearance of the reacting materials, it was concentrated under reduced pressure. The resultant was purified by silica gel column chromatography (chloroform : methanol =10 : 1) to obtain the desired compound 39 in an amount of 140 mg.

24-4. Preparation of chondroitin sulfate introduced with montelukast

560 mg of sodium chondroitin sulfate (weight average molecular weight of 25 kDa) was dissolved in 55 mL of distilled water and 55 mL of ethanol, and added with 140 mg of the compound 39. After that, it was added with 104 mg of 4-(4,6-dimethoxy-1,3,5-triazin-2-yl)-4-methylmorpholinium chloride n-hydrate followed by stirring overnight. Then, 420 mg of sodium hydrogen carbonate was added followed by stirring for 2 hours. Then, 225 μL of acetic acid and 1.7 g of sodium chloride were added in order thereto. After stirring for 30 minutes, 200 mL of 90% ethanol/distilled water was added for forming precipitates. The supernatant was discarded and washing with 90% ethanol/distilled water was performed 2 times. The obtained precipitates were dried overnight, and as a polysaccharide derivative, 530 mg of the desired compound xxiv was obtained. As a result of obtaining the introduction ratio by a carbazole sulfate method, it was found to be 11%.

(Example 25) Preparation of chondroitin sulfate introduced with ipratropium

25-1. Preparation of Boc-β-alanyl-ipratropium

46 mg of Boc-β-alanine was dissolved in 4 mL of dichloromethane and 4 mL of dimethyl formamide followed by addition of 100 mg of ipratropium and 82 mg of diisopropylethylamine and 1-hydroxybenzotriazole. After that, under ice cooling, 125 mg of dicyclohexylcarbodiimide was added followed by stirring overnight at room temperature. After confirming a new spot by thin layer chromatography, the reaction solution was with celite 1-hydroxybenzotriazole, liquid fractionation extraction was performed 3 times by using toluene and water. The collected aqueous layer was subjected to freeze drying to remove the solvent, and as a result, a crude product (240 mg) of the desired compound 40 was obtained.

25-2. Preparation of β-alanyl-ipratropium

240 mg of the compound 40 was dissolved in 7 mL of dichloromethane. Under ice cooling, 3 mL of 4 M hydrochloric acid/ethyl acetate was added and it was stirred for 1 hour. After that, the temperature was raised to room temperature, and it was stirred again for 2 hours. The reaction solution was concentrated under reduced pressure by using an evaporator in water bath at 40°C followed by washing with diethyl ether. As a result, 185 mg of a crude product of the desired compound 41 was obtained (i.e., a mixture of the compound 41,1-hydroxybenzotriazole, and ipratropium).

25-3. Preparation of chondroitin sulfate introduced with β-alanine-ipratropium

676 mg of sodium chondroitin sulfate (weight average molecular weight of 25 kDa) was dissolved in 10 mL of distilled water, and added with a mixture solution of ethanol (14 mL) and water (4 mL) containing 185 mg of the crude product of the compound 41. After that, 189 mg of 4-(4,6-dimethoxy-1,3,5-triazin-2-yl)-4-methylmorpholinium chloride n-hydrate was added thereto and stirred overnight. Subsequently, 676 mg of sodium chloride was added, and the solution was added dropwise to 90 mL of ethanol to form precipitates. The supernatant was discarded and washing with 90% ethanol/distilled water was performed 2 times. The obtained precipitates were dried overnight under reduced pressure, and as a polysaccharide derivative, 649 mg of the desired compound xxv was obtained. As a result of obtaining the introduction ratio by $^1$H-NMR, it was found to be about 16%.

(Example 26) Preparation of chondroitin sulfate introduced with tulobuterol

26-1. Preparation of Boc-β-alanyl-tulobuterol

Tulobuterol (1.00 g) and Boc-β-alanine (0.99 g) were dissolved in dichloromethane (10 mL). After cooling to 0°C, N,N-dimethylaminopyridine (161.3 mg) and water soluble carbodiimide (1.11 g) were added thereto followed by stirring for 3 hours at room temperature. After confirming the disappearance of the reacting materials by thin layer chromatography, a saturated aqueous solution of ammonium chloride was added to terminate the reaction. After dilution with ethyl acetate, the organic layer was washed with a saturated aqueous solution of ammonium chloride, a saturated aqueous solution of sodium hydrogen carbonate, and saturated brine. After drying over magnesium sulfate anhydrous, it was filtered and the solvent was distilled off under reduced pressure to obtain the desired compound 42 (1.71 g, 98%).

26-2. Preparation of β-alanyl-tulobuterol

1.71 g of the compound 42 was dissolved in 10 mL of tetrahydrofuran. After cooling to 0°C, 15 mL of 4 M hydrochloric acid/ethyl acetate was added and it was stirred for 2 hours at room temperature. After confirming the disappearance of the reacting materials by thin layer chromatography, the solvent was distilled off under reduced pressure to obtain the desired compound 43 (1.47 g, 92%).

26-3. Preparation of chondroitin sulfate introduced with tulobuterol

To 500 mg of chondroitin sulfate (weight average molecular weight of 20 kDa), 7.5 mL of distilled water was added and stirred for 30 minutes for dissolution. 7.5 mL of ethanol was slowly added and the solution was homogenously stirred. Thereafter, 74.3 mg of the compound 43 was dissolved in 3 mL solution of ethanol/distilled water = 1/1 and added thereto. Subsequently, 55.3 mg of 4-(4,6-dimethoxy-1,3,5-triazin-2-yl)-4-methylmorpholinium chloride n-hydrate was dissolved in 3 mL of ethanol/distilled water =1/1 solution and added thereto followed by stirring overnight. After adding 500 mg of sodium chloride, the reaction solution was added to 100 mL of 90% ethanol/distilled water to form precipitates. After allowing it to stand for a while, the supernatant was removed. Then, 100 mL of 90% ethanol/distilled water was added, stirred and washed for 5 minutes, and allowed again to stand for a while. The same washing was additionally performed 2 times, and after filtering through a glass filter and drying the obtained precipitate overnight at heating under reduced pressure conditions (40°C, 75 mmHg), the desired compound xxvi (455 mg) was obtained as a polysaccharide derivative. As a result of measuring $^1$H-NMR, the introduction ratio was found to be 10%.

(Example 28) Preparation of chitosan introduced with budesonide

28-1. Preparation of budesonide succinic acid ester

116 mg (1.0 eq., 1.16 mmol) of succinic anhydride was dissolved in 15 mL of tetrahydrofuran, and after being added with 500 mg (1.0 eq., 1.16 mmol) of budesonide and 28 mg (0.2 eq., 0.23 mmol) of N,N-dimethyl-4-aminopyridine, it was stirred overnight at room temperature. After confirming a new spot by thin layer chromatography, the reaction was terminated by adding, at 0°C, a saturated aqueous solution of ammonium chloride, and liquid fractionation extraction was performed 3 times by using ethyl acetate and 1 M hydrochloric acid. The collected organic layer was washed with a saturated aqueous brine solution. After drying over magnesium sulfate, it was concentrated under reduced pressure by using an evaporator in water bath at 40°C. The concentrate was purified by silica gel column chromatography (chloroform: methanol =15 : 1) to obtain the desired compound a in an amount of 615 mg.

28-2. Preparation of chitosan introduced with budesonide

900 mg of chitosan 10 (i.e., 0.5% chitosan solution dissolved in 0.5% acetic acid, viscosity: about 10 cps, manufactured by Wako Pure Chemical Industries, Ltd.) was dissolved in 50 ml of distilled water and 5 ml of 1 M hydrochloric acid. After that, 1 M sodium hydroxide solution was added dropwise thereto to have pH 5.6. After that, it was additionally stirred for 18 hours, and after being added with 593 mg (0.2 eq., 1.12 mmol) of the compound a and 524 mg (0.2 eq., 1.12 mmol) of 4-(4,6-dimethoxy-1,3,5-triazin-2-yl)-4-methylmorpholinium chloride n-hydrate, it was stirred overnight. Thereafter, 900 mg of sodium chloride was added. The resulting solution was added to 300 mL of 90% ethanol/distilled water to form precipitates. The supernatant was discarded. Washing with 90% ethanol/distilled water, washing with ethanol, and washing with acetone were performed 2 times for each (As a result of drying overnight the obtained precipitates under reduced pressure, 1.18 g of the target product xxviii was obtained.

<Drug dissociation test>

In the examples relating to drug dissociation test, the drug dissociation ratio was evaluated as described below.

(1) Preparation of phosphate buffer solution

Sodium dihydrogen phosphate dihydrate (780 mg (5.0 mmol)) was dissolved in 500 mL of distilled water to give a liquid A. Separately, disodium hydrogen phosphate dodecahydrate (1.79 g (5.0 mmol)) was dissolved in 500 mL of distilled water to give a liquid B. By mixing the liquid A and liquid B at ratio of 2 : 3, a phosphate buffer solution of pH 7.5 was prepared.

(2) Preparation of borate buffer solution

0.62 g (10 mmol) of boric acid and 0.75 g (10 mmol) of potassium chloride were weighed and dissolved in 500 mL of distilled water to give a liquid C. Separately, by diluting 0.1 N sodium hydroxide solution with distilled water (x 10 dilution), a liquid D was prepared.

50 mL of the liquid C and 7 mL of the liquid D were added to a 100 mL mess flask, and according to dilution to 100 mL with distilled water, a borate buffer solution of pH 8.0 was prepared. Furthermore, 50 mL of the liquid C and 40 mL of the liquid D were added to a 100 mL mess flask, and according to mess-up to 100 mL with distilled water, a borate buffer solution of pH 9.0 was prepared.

(3) Evaluation method

Each polysaccharide derivative obtained from above was dissolved in a buffer solution at each pH so as to have a concentration of 5 mg/10 mL, and heated for 1 week in an incubator at 36°C. Then, the drug dissociation ratio during the incubation was evaluated every 24 hours by liquid chromatography (HPLC).

The evaluation was performed by measuring the ratio between the drug amount remained in a polysaccharide derivative and the dissociated drug amount according to use of a size exclusion chromatography column (manufactured by TOSOH, TSKgel $\alpha$-6000, 7.8 mmI.D. $\times$ 30 cm).

Detailed conditions for the liquid chromatography are as follows.

Analysis time: 40 minutes
Flow rate: 0.5 mL/min
Gradient: isoclatic
Solution for elution: acetonitrile (for HPLC) : physiological saline = 1 : 2
Detector: UV detector (240 nm)
Temperature: 36°C

(Test example A1) Change in dissociation characteristics caused by different drug introduction ratio

The compounds ia and ib produced in Example 1 were evaluated by HPLC in terms of the drug dissociation ratio (%) for 1 week at 36°C. The evaluation of drug dissociation ratio was performed every 24 hours. The results are shown below.

[Table 3]

| Lapse of time (day) | Compound ia | | Compound ib | |
|---|---|---|---|---|
| | pH7.4 | pH8.0 | pH7.4 | pH8.0 |
| 0 | 0.0 | 0.0 | 0.5 | 0.6 |
| 1 | 0.8 | 2.9 | 1.2 | 2.9 |
| 2 | 1.5 | 5.2 | 1.7 | 4.8 |
| 3 | 2.3 | 7.7 | 2.4 | 6.7 |
| 4 | 3.2 | 10.6 | 3.2 | 9.4 |
| 5 | 4.0 | 12.8 | 3.8 | 11.4 |
| 6 | 4.6 | 14.8 | 4.4 | 13.2 |
| 7 | 5.3 | 16.7 | 5.0 | 15.0 |
| Dissociation ratio (%/day) | 0.8 | 2.4 | 0.7 | 2.1 |

The compound ia had dissociation ratio of 0.8%/day at pH 7.4 and 2.4%/day at pH 8.0. Meanwhile, the compound ib had dissociation ratio of 0.7%/day at pH 7.4 and 2.1 %/day at pH 8.0 so that the drug dissociation ratio was hardly affected by a change in drug introduction ratio.

(Test example A2) Change in dissociation characteristics caused by different polysaccharide

The compounds vb, vc, via, vib and xxa produced in Examples 5, 6, and 20 were evaluated in the same manner as above by HPLC in terms of the drug dissociation ratio (%) for 1 week at 36°C. The evaluation results are shown below.

[Table 4]

| Lapse of time (day) | Compound via | | Compound vib | | Compound vb | | Compound vc | | Compound xxa | |
|---|---|---|---|---|---|---|---|---|---|---|
| | pH7.4 | pH8.0 | pH7.4 | pH8.0 | pH7.4 | pH8.0 | pH7.4 | pH8.0 | pH 7.5 | pH 8.0 |
| 0 | 2.0 | 0.9 | 3.3 | 0.4 | 0.3 | 0.2 | 0.4 | 0.1 | 2.0 | 2.7 |
| 1 | 9.6 | 15.0 | 9.3 | 12.8 | 1.2 | 1.2 | 1.6 | 1.9 | 4.8 | 9.9 |
| 2 | 16.8 | 20.4 | 16.3 | 16.1 | 2.4 | 1.7 | 2.8 | 2.4 | 7.4 | 16.4 |
| 3 | 18.3 | 26.4 | 18.4 | 23.2 | 3.0 | 2.9 | 3.7 | 3.9 | 10.0 | 22.1 |
| 4 | 21.7 | 39.9 | 22.3 | 36.7 | 3.6 | 5.5 | 4.8 | 7.6 | 12.6 | 27.1 |
| 5 | 23.9 | 46.2 | 25.2 | 44.1 | 4.0 | 7.6 | 5.3 | 9.7 | 15.0 | 31.7 |
| 6 | 30.9 | 52.6 | 30.7 | 50.2 | 5.1 | 8.9 | 6.8 | 11.2 | 17.4 | 35.9 |
| 7 | 34.3 | 57.1 | 34.6 | 55.3 | 6.4 | 10.8 | 8.5 | 14.0 | 19.8 | 39.6 |
| Dissociation ratio (%/day) | 4.9 | 8.2 | 4.9 | 7.9 | 0.9 | 1.5 | 1.2 | 2.0 | 2.8 | 5.7 |

The dissociation ratios of the compounds via and vib, which are a hyaluronic acid derivative, were 4.9%/day and 4.9%/day, respectively, at pH 7.4 and 8.2%/day and 7.9%/day, respectively, at pH 8.0. The dissociation ratios of the compounds vb and vc, which are a chondroitin sulfate derivative, were 0.9%/day and 1.2%/day, respectively, at pH 7.4 and 1.5%/day and 2.0%/day, respectively, at pH 8.0. Furthermore, the dissociation ratio of the compound xxa, which is a carboxymethyl cellulose derivative, was 2.8%/day at pH 7.4 and 5.7%/day at pH 8.0. The drug was easily dissociated in the order of hyaluronic acid > carboxymethyl cellulose > chondroitin sulfate.

(Test example A3) Change in dissociation characteristics caused by different spacer

The compounds ix, x and xi were dissolved in a buffer solution at each pH so as to have 5 mg/10 mL (0.05 w/w%). It was then incubated for 1 week in an incubator at 36°C, during which time the drug dissociation ratio (%) was evaluated in the same manner as above. The evaluation results are shown below.

[Table 5]

| Lapse of time (day) | Compound ix | | Compound x | | Compound xi | |
|---|---|---|---|---|---|---|
| | pH7.4 | pH8.0 | pH7.4 | pH8.0 | pH7.4 | pH8.0 |
| 0 | 0.4 | 1.7 | 1.1 | 4.3 | 0.5 | 0.0 |
| 1 | 2.0 | 6.3 | 5.7 | 14.4 | 0.2 | 0.5 |
| 2 | 3.6 | 10.9 | 9.3 | 23.5 | 0.2 | 0.7 |
| 3 | 5.3 | 16.1 | 11.0 | 29.3 | 0.2 | 1.1 |
| 4 | 8.7 | 21.7 | 15.4 | 37.8 | 1.5 | 2.8 |
| 5 | 11.0 | 26.1 | 19.6 | 44.7 | 2.1 | 3.8 |
| 6 | 12.2 | 28.3 | 22.1 | 48.7 | 1.9 | 4.3 |
| 7 | 11.5 | 29.3 | 22.0 | 50.6 | 0.6 | 2.8 |
| Dissociation ratio (%/day) | 1.6 | 4.2 | 3.1 | 7.2 | - | - |

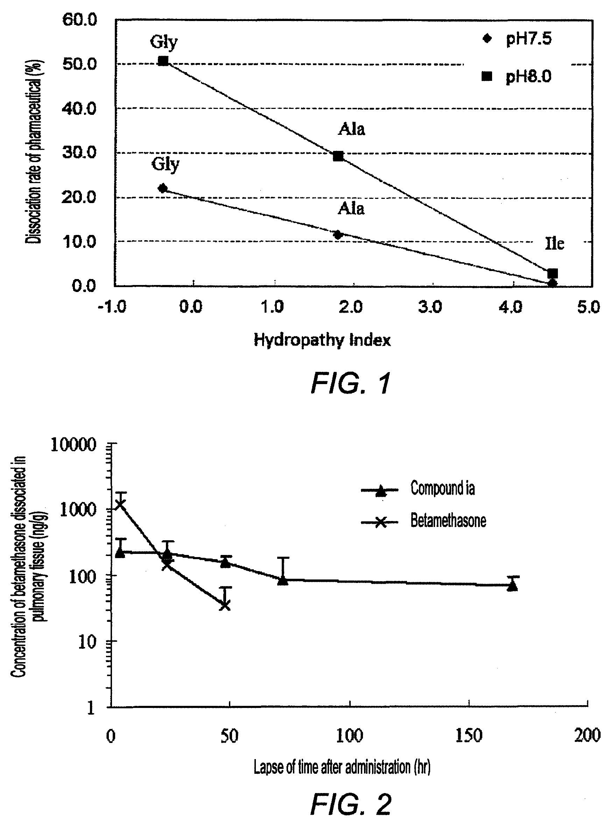

The dissociation rate of the compound x (CS-Gly-BTM) was 3.1%/day at pH 7.4 and 7.2%/day at pH 8.0. The dissociation rate of the compound ix (CS-Ala-BTM) was 1.6%/day at pH 7.4 and 4.2%/day at pH 8.0.
The correlation between hydropathy index and dissociation rate of each amino acid was evaluated. The results are shown in Fig. 1.
The correlativity was observed at any pH. This result indicates that, by evaluating the hydropathy index of a spacer, it is possible to estimate and control the dissociation rate.
It is expected that, when a natural amino acid is used as a spacer, isoleucine maintains the drug efficacy for the longest time period as it has the strongest hydrophobic property (i.e., the hydropathy index value is the highest).

Thus, it is found that, if it is desired to further suppress the dissociation rate, a modification for increasing the hydrophobic property may be performed for the structure of isoleucine or an amino carboxylic acid with higher hydropathy index may be used.

(Test example A4) Change in release properties according to introduction at position 21 or introduction at position 11.

The compounds ia, va, vii, viii, and xvii prepared in Examples 1, 5, 7, 8, and 17 were dissolved in a buffer solution at each pH so as to have 5 mg/10 mL. It was then incubated for 1 week in an incubator at 36°C, during which time the drug dissociation ratio was evaluated in the same manner as above. The evaluation results are shown below.

[Table 6]

| Lapse of time (day) | Compound ia | | Compound va | | Compound viii | | Compound viii | | Compound xvii | |
|---|---|---|---|---|---|---|---|---|---|---|
| | pH7.4 | pH8.0 | pH7.4 | pH8.0 | pH7.4 | pH8.0 | pH7.4 | pH8.0 | pH7.4 | pH8.0 |
| 0 | 0.0 | 0.0 | 0.0 | 0.0 | 0.0 | 0.0 | 0.1 | 0.3 | 0.0 | 0.0 |
| 1 | 0.8 | 2.9 | 0.4 | 2.3 | 0.0 | 0.3 | 7.9 | 14.8 | 0.3 | 4.6 |
| 2 | 1.5 | 5.2 | 1.0 | 4.2 | 0.0 | 0.6 | 4.9 | 26.3 | 0.7 | 10.1 |
| 3 | 2.3 | 7.7 | 1.8 | 7.7 | 0.2 | 0.7 | 12.7 | 27.1 | 1.3 | 14.1 |
| 4 | 3.2 | 10.6 | 3.1 | 10.3 | 0.4 | 0.7 | 8.4 | 29.8 | 1.9 | 17.8 |
| 5 | 4.0 | 12.8 | 3.8 | 12.8 | 0.4 | 1.0 | 15.2 | 64.5 | 2.3 | 18.9 |
| 6 | 4.6 | 14.8 | 4.4 | 14.0 | 0.4 | 1.0 | 14.3 | 47.4 | 2.5 | 20.0 |
| 7 | 5.3 | 16.7 | 5.3 | 17.5 | 0.4 | 1.1 | 21.8 | 70.9 | 2.9 | 22.8 |
| Dissociation ratio (%/day) | 0.8 | 2.4 | 0.8 | 2.5 | 0.1 | 0.2 | 3.1 | 10.1 | 0.4 | 3.3 |

When comparison was made between the compounds ia and va in which a steroid had been introduced through the hydroxyl group at position 21, it was found that the same dissociation rate is shown regardless of a difference in type of drugs.

$$\text{CS-}\beta\text{Ala-BET } 0.8 \text{ (pH 7.4)/2.4 (pH 8.0) (ia)}$$

$$\text{CS-}\beta\text{Ala-BUD } 0.8 \text{ (pH 7.4)/2.5 (pH 8.0) (va)}$$

Meanwhile, the compound vii (CS-βAla-FP) in which a steroid had been introduced through the hydroxyl group at position 11 exhibited dissociation rate of 0.1 %/day at pH 7.4 and 0.2%/day at pH 8.0, and drug dissociation was hardly observed.
From the above results, it was shown that the dissociation rate was the same if the structure of drug is similar and the modification site is the same while a different dissociation rate was exhibited according to a difference in modification site in drug.
In this regard, even for a compound in which a steroid has been introduced through the hydroxyl group at position 11, the dissociation ratio of the compound xvii (CS-Gly-FP) in which glycine with low hydropathy index value was used as a spacer was 0.4%/day at pH 7.4 and 3.3%/day at pH 8.0, and the dissociation ratio of the compound viii (CS-2F βAla-FP) in which a halogen-introduced spacer with increased electron withdrawing property for an ester bond was used was 3.1 %/day at pH 7.4 and 10.1%/day at pH 8.0, indicating that the dissociation rate was accelerated by both spacers. In terms of the solvent composition, for the drug dissociation test for the compound xvii, phosphate buffer solution/acetonitrile (1 : 1) was used for pH 7.4 and borate buffer solution/acetonitrile (1 : 1) was used for pH 8.0.

(Test example A5) Comparison of dissociation ratio of polysaccharide derivative introduced with β-alanyl-proxyphylline

The compounds xxiia and xxiib were dissolved in a buffer solution at each pH to have 5 mg/10 mL (0.05 w/w%). It was then incubated for 1 week in an incubator at 36°C, during which time the drug dissociation ratio was evaluated in the same manner as above. The evaluation results are shown below.

[Table 7]

| Lapse of time (day) | Compound xxiia | | Compund xxiib | |
| --- | --- | --- | --- | --- |
| | pH7.4 | pH8.0 | pH7.4 | pH8.0 |
| 0 | 0.0 | 0.0 | 0.0 | 0.0 |
| 1 | 0.0 | 0.0 | 0.7 | 2.1 |
| 2 | 0.0 | 0.0 | 1.3 | 4.6 |
| 3 | 0.0 | 1.2 | 2.2 | 7.0 |
| 4 | 0.0 | 1.6 | 2.8 | 8.8 |
| 5 | 0.0 | 2.0 | 3.6 | 10.4 |
| 6 | 0.8 | 2.3 | 4.4 | 12.6 |
| 7 | 1.3 | 2.6 | 5.3 | 14.3 |
| Dissociation ratio (%/day) | 0.2 | 0.4 | 0.8 | 2.0 |

The compound xxiia exhibited dissociation rate of 0.2%/day at pH 7.4 and 0.4%/day at pH 8.0. The compound xxiib exhibited dissociation rate of 0.8%/day at pH 7.4 and 2.0%/day at pH 8.0.

(Test example A6) Dissociation ratio of GAG introduced with seratrodast-2-aminoethyl ester

The compound xxiii was dissolved in a buffer solution at each pH to have 0.05%. It was then incubated for 1 week in an incubator at 36°C, during which time the drug dissociation ratio was evaluated in the same manner as above. The evaluation results are shown below.

[Table 8]

| Lapse of time (day) | Compound xiii | |
| --- | --- | --- |
| | pH7.4 | pH8.0 |
| 0 | 0.0 | 0.0 |
| 1 | 0.0 | 0.2 |
| 2 | 0.2 | 0.4 |
| 3 | 0.3 | 0.6 |
| 4 | 0.3 | 0.7 |
| 5 | 0.4 | 0.9 |
| 6 | 0.5 | 1.1 |
| 7 | 0.6 | 1.3 |
| Dissociation ratio (%/day) | 0.1 | 0.2 |

The compound xxiii exhibited dissociation rate of 0.1%/day at pH 7.4 and 0.2%/day at pH 8.0.

(Test example A8) Comparison of dissociation ratio of CMC-βAla-BUD

The compound xxa was dissolved in a buffer solution at each pH to have 5 mg/30 mL (0.05 w/w%). It was then incubated for 1 week in an incubator at 36°C, during which time the drug dissociation ratio was evaluated in the same manner as above. The evaluation results are shown below.

[Table 9]

| Lapse of time (day) | Compound xxa | |
|---|---|---|
| | pH 7.5 | pH 8.0 |
| 0 | 2.0 | 2.7 |
| 1 | 4.8 | 9.9 |
| 2 | 7.4 | 16.4 |
| 3 | 10.0 | 22.1 |
| 4 | 12.6 | 27.1 |
| 5 | 15.0 | 31.7 |
| 6 | 17.4 | 35.9 |
| 7 | 19.8 | 39.6 |
| Dissociation ratio (%/day) | 2.8 | 5.7 |

As a result, it was found that the dissociation rate was 2.6%/day at pH 7.4 and 5.5%/day at pH 8.0.

(Test example A9) Dissociation test for ornithine-introduced CS

The compound xiii was dissolved in a buffer solution at each pH to have 5 mg/30 mL (0.05 w/w%). It was then incubated for 1 week in an incubator at 36°C, during which time the drug dissociation ratio was evaluated in the same manner as above. The evaluation results are shown below.

[Table 10]

| Lapse of time (day) | Compound xiii | |
|---|---|---|
| | pH7.5 | pH8.1 |
| 0 | 0.5 | 0.7 |
| 1 | 4.8 | 7.5 |
| 2 | 6.6 | 11.2 |
| 3 | 8.5 | 14.1 |
| 4 | 9.7 | 17.2 |
| 5 | 10.8 | 19.7 |
| 6 | 12.2 | 21.8 |
| 7 | 13.2 | 23.9 |
| Dissociation ratio (%/day) | 1.9 | 3.4 |

The compound xiii exhibited dissociation rate of 1.8%/day at pH 7.4 and 3.3%/day at pH 8.0.

(Test example A10) Release of betameta-CS

The compound xxi was dissolved in a buffer solution at each pH to have 5 mg/30 mL (0.05 w/w%). It was then incubated for 1 week in an incubator at 36°C, during which time the drug dissociation ratio was evaluated in the same manner as above. The evaluation results are shown below.

[Table 11]

| Lapse of time (day) | Compound xxi | |
|---|---|---|
| | pH7.5 | pH8.1 |
| 0 | 0.0 | 0.0 |

(continued)

| Lapse of time (day) | Compound xxi | |
|---|---|---|
| | pH7.5 | pH8.1 |
| 1 | 5.1 | 7.2 |
| 2 | 7.7 | 10.2 |
| 3 | 10.1 | 13.8 |
| 4 | 12.2 | 17.0 |
| 5 | 14.2 | 20.8 |
| 6 | 16.3 | 23.2 |
| 7 | 18.2 | 25.4 |
| Dissociation ratio (%/day) | 2.6 | 3.6 |

The dissociation ratio was 2.7%/day at pH 7.4 and 3.9%/day at pH 8.0.

(Test example A11) Release data of CS-βAla-Pred

The compound xiv was dissolved in a buffer solution at each pH to have 5 mg/30 mL (0.05 w/w%). It was then incubated for 1 week in an incubator at 36°C, during which time the drug dissociation ratio was evaluated in the same manner as above. The evaluation results are shown below.

[Table 12]

| Lapse of time (day) | Compound xiv | |
|---|---|---|
| | pH7.5 | pH8.1 |
| 0 | 0.3 | 0.3 |
| 1 | 3.4 | 8.2 |
| 2 | 5.7 | 14.3 |
| 3 | 8.1 | 20.4 |
| 4 | 10.4 | 26.1 |
| 5 | 12.4 | 29.8 |
| 6 | 14.5 | 34.4 |
| 7 | 16.3 | 38.0 |
| Dissociation ratio (%/day) | 2.3 | 5.4 |

The dissociation rate was 2.3%/day at pH 7.4 and 5.4%/day at pH 8.0.

<In vivo evaluation>

(Test example B1) Evaluation of persistent property of drug in pulmonary tissue and blood kinetics in normal rat

B1-1. Evaluation of persistent property in pulmonary tissue

As a test substance, the compound ia was used, and it was used after being dissolved in phosphate buffered saline such that the pharmaceutical concentration is 3 mg/mL. Furthermore, as a control, betamethasone phosphate was used after it was suspended in phosphate buffered saline to have concentration of 3 mg/mL.

For the test, a BN/CrlCrlj rat (Charles River Laboratories International, Inc., 5-week old) was used. It was placed on its back and fixed under general anesthesia, and by using a sonde for intratracheal administration (MicroSprayer (registered trademark) Aerosolizer Model IA-1B, manufactured by Penn-Century), intratracheal administration was performed with volume of 100 μL. As general anesthesia, inhalation of isoflurane (concentration of 3.0% and flow rate of 2.0 L/min) was employed.

At Hour 4, 24, 48, 72 and 168 after administration of a test substance, the pulmonary tissue was collected under pentobarbital anesthesia. Weight of the pulmonary tissue was measured and, after being added with an aqueous solution of ammonium formic acid (pH 6.0) : methanol (3 : 2, v/v) at ratio of 40x (1 g: 40 mL), it was homogenated for about 1 minute under ice cooling by using a homogenizer. Subsequently, the obtained pulmonary tissue homogenate was extracted with chloroform and methanol and, by using a liquid chromatograph-tandem mass analyzer, content of dissociated betamethasone in the pulmonary tissue was measured. Meanwhile, the measurement was made with n = 3 for each evaluation time point.

From the obtained betamethasone content, parameters of pharmacokinetics were calculated. Each parameter of pharmacokinetics was calculated from a change in average content in pulmonary tissue. If there was an animal showing the measurement value of less than the lower limit of quantification, the average content in pulmonary tissue was calculated by substituting the value of 0 (zero) for it. The time t (hr) for calculating $AUC_{0-t}$ was the final time point at which the quantification can be made. Meanwhile, for calculation of the parameters of pharmacokinetics, non-compartmental analysis model analysis by WinNonlin Professional (registered trademark) Ver 5.2.1 was used. As a comparison, the same evaluation as above was performed for a case in which betamethasone alone was used instead of the compound ia. The results are shown in Fig. 2 and the following table.

[Table 13]

| Compound | $C_{max}$ (ng/g) | $T_{max}$ (hr) | $AUC_{0-t}$ (ng·h/g) | $AUC_{0-\infty}$ (ng·h/g) | $T_{1/2}$ (hr) |
|---|---|---|---|---|---|
| Compound ia | 218 | 4 | 19295 | 31988 | 128 |
| Betamethasone | 1189 | 4 | 21371 | 21797 | 8.7 |

When intratracheal administration of the compound ia was performed for a normal rat, exposure of betamethasone was observed in the pulmonary tissue until 168 hours after the administration, which is the final evaluation time point. Meanwhile, betamethasone alone exhibited the exposure only until 48 hours after the administration (lower limit for quantification: 10 ng/g). The disappearance half life of the betamethasone after intratracheal administration of the compound ia to a normal rat was 128.0 hours. Meanwhile, the disappearance half life of the betamethasone content in pulmonary tissue after intratracheal administration of betamethasone alone was 8.7 hours.

B1-2. Evaluation of blood kinetics

Next, a change in plasma drug concentration was evaluated.

Blood (300 μL) was collected, at Hour 0.5, 1, 2, 4, 8, 24, 48, 72 and 168 after administration of the compound ia and betamethasone, from a jugular vein by using a disposable syringe attached with 27 G needle treated with heparin, and the blood was rapidly centrifuged (1800×g, 4°C, 15 min). The obtained plasma (100 μL) was added with a mixed solution of 900 μL of an aqueous solution of ammonium formic acid (pH 6.0): methanol (1 : 2, v/v), and stirred. The resultant was extracted with chloroform and methanol, and the extracted solution was measured by LC-MS/MS.

(Parameters of pharmacokinetics)

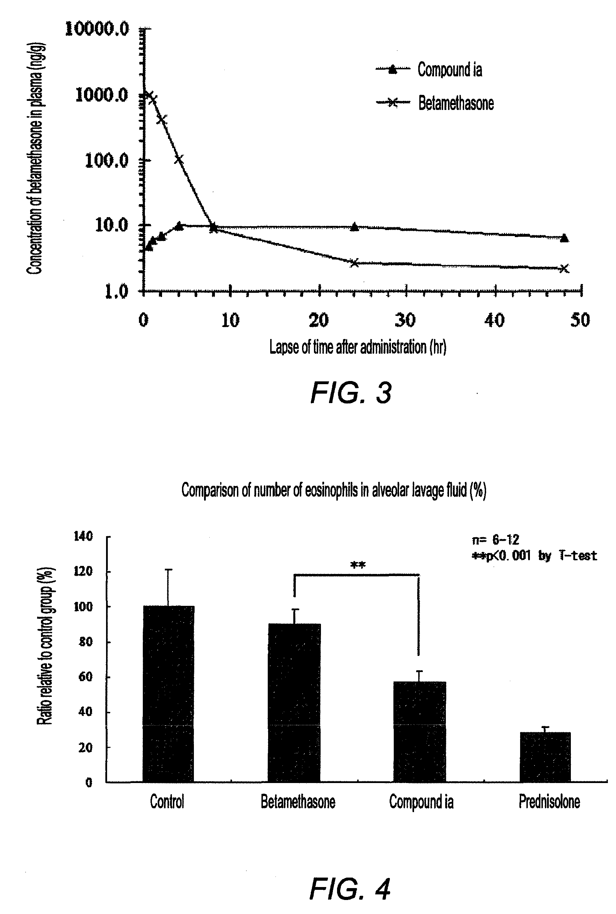

From the obtained plasma concentration of betamethasone, parameters of pharmacokinetics were calculated. Each parameter of pharmacokinetics was calculated from a change in average content in plasma. If there was an animal showing the measurement value of less than the lower limit of quantification (< 5 ng/mL), the average content in plasma was calculated by substituting the value of 0 (zero) for it. Meanwhile, for calculation of the parameters of pharmacokinetics, non-compartmental analysis model analysis by WinNonlin Professional Ver 5.2.1 was used. The results are shown in Fig. 3 and the following table.

[Table 14]

| Compound | $C_{max}$ (ng/g) | Tmax (hr) | $T_{1/2}$(hr) |
|---|---|---|---|
| Compound ia | 10.1 | 4 | 146.9 |
| Betamethasone | 1008.9 | 0.5 | 1.1 |

After administration of betamethasone alone, the plasma concentration rapidly increased followed by gradual decrease, and after 24 hours, it was lower than the lower limit for quantification. On the other hand, in case of the compound ia, the plasma concentration gradually increased and stable plasma concentration was maintained over 48 hours.

(Test example B2) Effect of betamethasone-introduced CS in rat antigen-induced asthma model

As for the test substance, phosphate buffered saline was used as a negative control, and as a positive control, 0.6 mg/mL prednisolone suspension was used and 2.5% aqueous solution of the compound ia and 3 mg/mL betamethasone suspension were used.

For the evaluation, a BN/CrlCrlj rat (Charles River Laboratories International, Inc., 5-week old) was used. Intraperitoneal administration of 1 mL of aluminum hydroxide gel containing ovalbumin (1 mg/mL) was performed 3 times with one day interval. 14 Days after performing the first sensitization, intraperitoneal administration of 1 mL of aluminum hydroxide gel containing ovalbumin (1 mg/mL) was performed again followed by the second sensitization. 7 Days after the second sensitization, inhalation exposure of physiological saline (10 mg/mL) containing ovalbumin was performed by using a nebulizer five times with one day interval in order to induce asthma.

For the administration of phosphate buffered saline, an aqueous solution of the compound ia, and betamethasone suspension, a rat was placed on its back and fixed under general anesthesia, and by using a sonde for intratracheal administration (MicroSprayer (registered trademark) Aerosolizer Model IA-1B, manufactured by Penn-Century), intratracheal administration was performed with volume of 100 μL. As general anesthesia, inhalation of isoflurane (concentration of 3.0% and flow rate of 2.0 L/min) was employed.

The 0.6 mg/mL prednisolone suspension was orally administered by using a sonde for oral administration under no anesthesia to have prednisolone amount of 3 mg/kg.

The phosphate buffered saline, an aqueous solution of the compound ia, and betamethasone suspension were administered only once, two days before inducing asthma (i.e., 7 days before the evaluation time point) while the prednisolone suspension was administered five times, every 30 minutes before inducing asthma (i.e., last administration was on the day before the evaluation time point). Meanwhile, the test was performed with n = 6 to 12.

24 Hours after the fifth exposure, phosphate buffered saline (2 mL) containing 0.1% bovine serum albumin heated to 37°C was injected to a bronchus of a rat under pentobarbital anesthesia and collected thereafter. This procedure was repeated 5 times to have bronchoalveolar lavage fluid. The bronchoalveolar lavage fluid was centrifuged for 3 minutes at 200 x g and room temperature. The supernatant was discarded, and the pellet was re-suspended in phosphate buffered saline (500 μL) containing 0.1 % bovine serum albumin.

Subsequently, a smear sample of the suspension was prepared followed by Diff-Quik staining. According to cell fractionation, ratio of eosinophil in white blood cells was calculated. According to integration with the number of white blood cells in bronchoalveolar lavage fluid, the number of neutrophil in bronchoalveolar lavage fluid was calculated. Meanwhile, the number of white blood cells in bronchoalveolar lavage fluid was measured by using a blood cell counting chamber. The results are shown in Fig. 4 and the following table.

[Table 15]

| | Control | % of control | | |
| --- | --- | --- | --- | --- |
| | | Betamethasone | Compound ia | Prednisolone |
| Average value | 100 | 90.0 | 56.8 | 28.0 |
| Standard deviation | 21.3 | 8.3 | 6.5 | 3.7 |

Compared to the group administered with phosphate buffered saline, the group administered with betamethasone showed no influence on eosinophil infiltration on Day 7 after the administration. However, the group administered with the compound ia showed a significantly suppressed eosinophil infiltration even on Day 7 after the administration, demonstrating the long-acting pharmaceutical effect.

Namely, the polysaccharide derivative of the present invention showing a persistent property in pulmonary tissue was confirmed to exhibit a long-acting pharmaceutical effect.

(Test example B3) Pharmacokinetics data

The compounds va, vb, vc, via, vib, xxa, and xxb which have been obtained from Examples 5, 6, and 20 were evaluated in the same manner as the method described in (Test example B1) in terms of the persistent property of the pharmaceutical in a pulmonary tissue of a normal rat. Meanwhile, the evaluation time points include three points, i.e.,

Hour 24, 72, and 168. The results are shown in the following table.

[Table 16]

| | Concentration in pulmonary tissue (ng/g) | | |
|---|---|---|---|
| | 24 hr | 72 hr | 168 hr |
| Compound va | 21.9 | 16.6 | 4.6 |
| Compound vb | 50.1 | 16.3 | 9.6 |
| Compound vc | 93.5 | 58.6 | 15.3 |
| Compound iva | 67.0 | 41.1 | 15.7 |
| Compound ivb | 152.7 | 51.4 | 22.4 |
| Compound xxa | 42.5 | 15.1 | 10.9 |
| Compound xxa | 35.2 | 17.2 | 4.3 |

From any of the test compounds, the persistence of the pharmaceutical preparation (lower limit of quantification: 2 ng/g) was observed after Hour 168. No significant difference in tissue persistence was observed according to a difference in polysaccharide (hyraluronic acid or chondroitin sulfate) (glycosaminoglycan or homnoglycan). However, when comparison is made between the same types of polysaccharides, there was a tendency that smaller molecular weight yields a higher pulmonary tissue concentration value than the larger molecular weight.

(Test example B4) Pharmacokinetics data

The compounds va, x, and xi which have been obtained from Examples 5,10, and 11 were evaluated in the same manner as the method described in (Test example B1) in terms of the persistent property of the pharmaceutical in a pulmonary tissue of a normal rat. Meanwhile, the evaluation time points include three points, i.e., Hour 24, 72, and 168. The results are shown in the following table.

[Table 17]

| | Concentration in pulmonary tissue (ng/g) | | |
|---|---|---|---|
| | 24 hr | 72 hr | 168 hr |
| Compound va | 50.1 | 16.3 | 9.6 |
| Compound xv | 119.8 | 99.9 | 37.3 |
| Compound xvi | 24.8 | 8.9 | 7.2 |

From all the test substances, exposure to pharmaceutical preparation was observed after Hour 168 (lower limit of quantification: 2 ng/g). The results show that use of glycine as a spacer yields the high concentration while use of isoleucine as a spacer yields the lowest concentration.

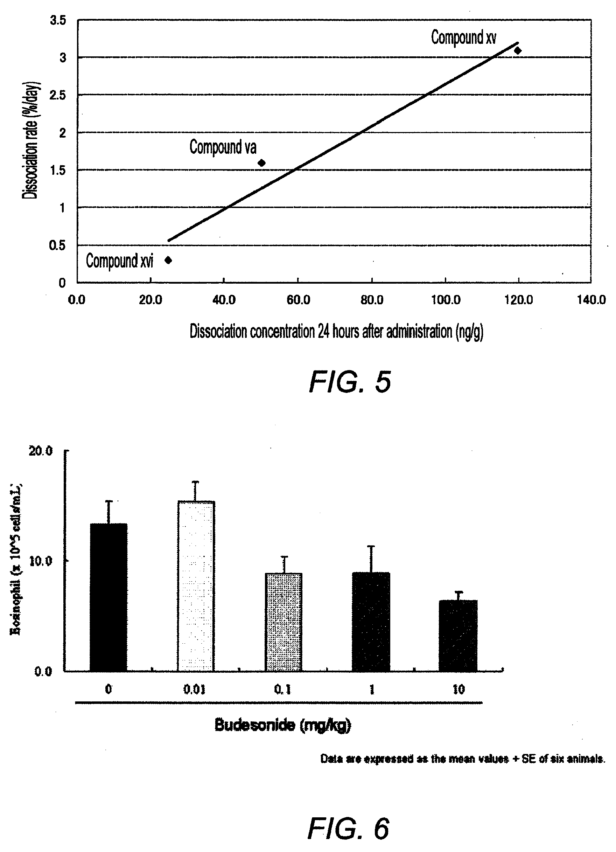

Furthermore, as shown in Fig. 5, the correlativity was shown between the dissociation ratio of each compound and dissociated drug concentration 24 hours after the administration.

(Test example B5) Pharmacokinetics data

The compounds va and xviiib which have been obtained from Examples 5 and 18 were evaluated in the same manner as the method described in (Test example B1) in terms of the persistent property of the pharmaceutical in a pulmonary tissue of a normal rat. Meanwhile, the evaluation time points include three points, i.e., Hour 24, 72, and 168. The results are shown in the following table.

[Table 18]

|  | Concentration in pulmonary tissue (ng/g) | | |
|---|---|---|---|
|  | 24 hr | 72 hr | 168 hr |
| Compound va | 50.1 | 16.3 | 9.6 |
| Compound xviiib | 111.4 | 16.4 | 0.6 |

Concentration of the compound va was maintained in a pulmonary tissue after 168 hours. On the other hand, the compound xviiib showed the value that is lower than the lower detection limit.

Both the compound va and the compound xviiib have good solubility in water, but as the compound va maintained the pulmonary tissue concentration even 168 hours after the administration, the persistent property in a pulmonary tissue was found to be improved due to the covalent bond to polysaccharide.

(Test example B6) Comparison of pharmacokinetics data between mixture with CS and single preparation

The compounds vb obtained from Example 5 was evaluated in the same manner as the method described in (Test example B1) in terms of the persistent property of the pharmaceutical in a pulmonary tissue of a normal rat.

As a comparative control group, budesonide alone in the same amount as the compound vb, and a mixture of budesonide and chondroitin sulfate in the same amount as the compound vb were administered. Meanwhile, in the present measurement system, the lower detection limit of budesonide was 1 ng/mL. Meanwhile, the evaluation time points include three points, i.e., Hour 4, 24, and 72. The results are shown in the following table.

[Table 19]

|  | Concentration in pulmonary tissue (ng/g) | | |
|---|---|---|---|
|  | 4 hr | 24 hr | 72 hr |
| Compound vb | - | 50.1 | 16.3 |
| Single preparation | 471 | 0.3 | 0.1 |
| Mixture | 271 | 1.7 | 0.5 |

On Hour 72 after the administration, the compound vb maintained budesonide concentration in a pulmonary tissue. However, from the budesonide alone and mixture of budesonide and chondroitin sulfate, the budesonide concentration in a pulmonary tissue was observed to be lower than the lower detection limit.

(Test example B7) Determination of pharmaceutically effective dose of budesonide

Dose responsiveness of budesonide alone in an OVA sensitized rat model was determined.

There were four doses for determination, i.e., 0.01 mg/kg, 0.1 mg/kg, 1 mg/kg, and 10 mg/kg.

As a test system, a male BN rat was used. For OVA sensitization, 1 mL of Alum containing 1 mg/mL OVA was administered intraperitoneally. After the sensitization, physiological saline containing 10 mg/mL OVA was used for inhalation exposure, 5 times every 24 hours, by using a nebulizer. The test substance was administered to a bronchus 1 hour before the 4th and 5th inhalation exposure of OVA.

On Hour 24 after the final exposure, 2 mL of PBS containing 0.1% BSA (heated to 37°C) was administered under pentobarbital anesthesia to a bronchus followed by recovery. This procedure was repeated 5 times to give bronchoalveolar lavage fluid (BALF).

BALF was centrifuged for 3 minutes at 200 x g and room temperature. The supernatant was discarded, and the pellet was re-suspended in PBS (500 $\mu$L, heated to 37°C) containing 0.1% BSA. Subsequently, a smear sample of the BALF was prepared followed by Diff-Quik staining. According to cell fractionation, ratio of eosinophil in white blood cells was calculated. According to integration with the number of white blood cells in BALF, the number of neutrophil in BALF was calculated (number of white blood cells was measured by using blood cell counting chamber).

The results are shown in Fig. 6. From the budesonide administration group, the inhibitory activity was shown at administration amount of 0.1 mg/kg or more. Since the activity was observed with dose responsiveness, the test dose was set at 1 mg/kg.

(Test example B8) Determination of pharmaceutically effective dose of budesonide

Dose responsiveness of the compound va in an OVA sensitized rat model was determined in the same manner as (Test example B2).

There were three doses for determination, i.e., 0.35 mg/kg, 3.5 mg/kg, and 35 mg/kg, such that the budesonide amount for administration is 0.1 mg/kg, 1 mg/kg, or 10 mg/kg.

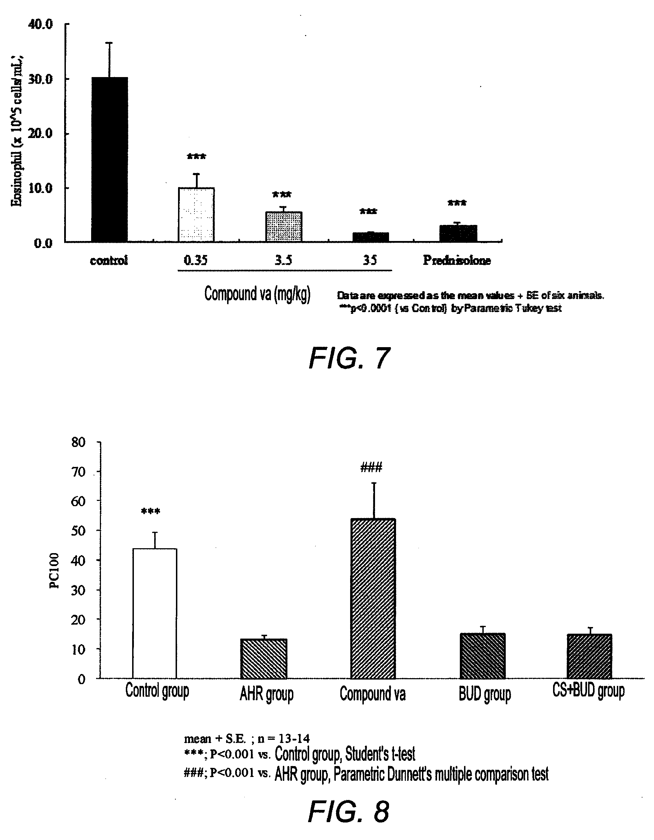

The results are shown in Fig. 7. Even at the administration amount of 0.35 mg/kg (i.e., 0.1 mg/kg in budesonide amount), the eosinophil-inhibiting activity was shown 1 week after the administration. Furthermore, the activity was observed with dose responsiveness.

(Test example B9) Effect of budesonide-introduced CS in mouse antigen-induced airway hypersensitivity model

As for the test substance, 2 mg/mL budesonide suspension, and a mixture of 2 mg/mL budesonide and 14.6 mg/mL chondroitin sulfate were used as a comparative control, and 1.6% solution of the compound va was used.

For the evaluation, BALB/cCrSlc (Japan SLC, Inc., 11-week old) was used. Intraperitoneal administration of 0.1 mL of ovalbumin-containing aluminum hydroxide and magnesium hydroxide gel (0.2 mg/mL) was performed 2 times with two week interval for sensitization. On Day 7,8, and 9 after the second sensitization, physiological saline containing ovalbumin (10 mg/mL) was used for inhalation exposure for 20 minutes by using a nebulizer. In addition, on Day 12 after the second sensitization, physiological saline containing ovalbumin (50 mg/mL) was used for inhalation exposure for 20 minutes by using a nebulizer in order to induce an asthma reaction.

Intratracheal administration (volume: 40 μL) of the budesonide suspension, mixture of budesonide and chondroitin sulfate, and an aqueous solution of the compound va was performed by using a glass microsyringe (Ito Micro Syringe, manufactured by ITO SEISAKUSHO CO., LTD.) after fixing the mouse on its back under general anesthesia. As general anesthesia, inhalation of isoflurane (concentration of 3.0% and flow rate of 1.0 L/min) was employed. The administration was performed only once, and it was performed 6 days after the second sensitization. Meanwhile, the test was performed with n = 6.

Airway hyper responsiveness (AHR) evaluation was performed as described below. On Day 13 after the second sensitization, the mouse was forced to inhale metacholine (hereinbelow, MCh) of 0, 5, 10 and 20 mM, and the airway resistance parameter (Penh) at 3 minutes after the inhalation of metacholine with different concentrations was measured by a device for measuring unrestrained respiratory function (manufactured by Buxco Research System). AUC value of the Penh value was measured, and a graph of MCh concentration vs. AUC was established. Then, based on the exponential approximation equation of the graph of MCh concentration vs. AUC, the estimated MCh concentration at which the AUC value is twice the estimated value for inhalation of physiological saline (i.e., PC100: Provocative Concentration of producing 100% increase in Penh) was calculated. The results are shown in Fig. 8.

According to the administration of the compound va 1 week before the evaluation, the enhanced sensitivity to MCh, which is observed from AHR group with induced asthma reaction, was inhibited to the level of the control group which has been undergone with the sensitization but not induced to have an asthma reaction. Meanwhile, no inhibition was observed from the group administered with budesonide suspension (BUD group) or the group administered with a mixture of budesonide and chondroitin sulfate (CS+BUD group) (Fig. 8). Namely, it was confirmed that, the compound va has a long-acting effect not only for anti-inflammatory reaction in a rat model with antigen-induced asthma but also for augmented airway hyper responsiveness.

(Test example B10) Effect of budesonide-introduced CS in mouse model having antigen-induced rhinitis

As a test substance, 2.7% solution of the compound va was used. As a negative control, phosphate buffered saline was used. As a comparative control, 4 mg/mL budesonide suspension was used.

For the evaluation, a BALB/cAnNCrlCrlj mouse (Charles River Laboratories International, Inc., 5-week old) was used and intraperitoneal administration of ovalbumin-containing aluminum hydroxide and magnesium hydroxide gel (0.5 mg/mL) to the mouse was performed 3 times with 1 week interval, 200 μL for each, for sensitization with ovalbumin. From Week 1 after the final administration of ovalbumin-containing aluminum hydroxide and magnesium hydroxide gel, the mouse sensitized with ovalbumin was subjected to intranasal administration of 20 μL of ovalbumin-containing physiological saline (40 mg/mL) with 24 hour interval to have nasal cavity sensitization · induction of 5 times. From Day 1 after the final nasal cavity sensitization · induction, 20 μL of ovalbumin physiological saline (40 mg/mL) was intranasally administered with 24 hour interval to induce a rhinitis reaction. The induction of the rhinitis reaction was performed 8 times in total for 8 days.

For the administration of the phosphate buffered saline, aqueous solution of the compound va, and budesonide suspension, the mouse was placed on its back and subjected to intranasal administration using micropipettor with dose of 10 μL for each nostril, i.e., 20 μL for both nostrils.

The phosphate buffered saline, aqueous solution of the compound va, and budesonide suspension were administered only once on the day of inducing initial rhinitis reaction, and 1 hour before administering physiological saline containing ovalbumin. Meanwhile, the test was basically performed with n = 6.

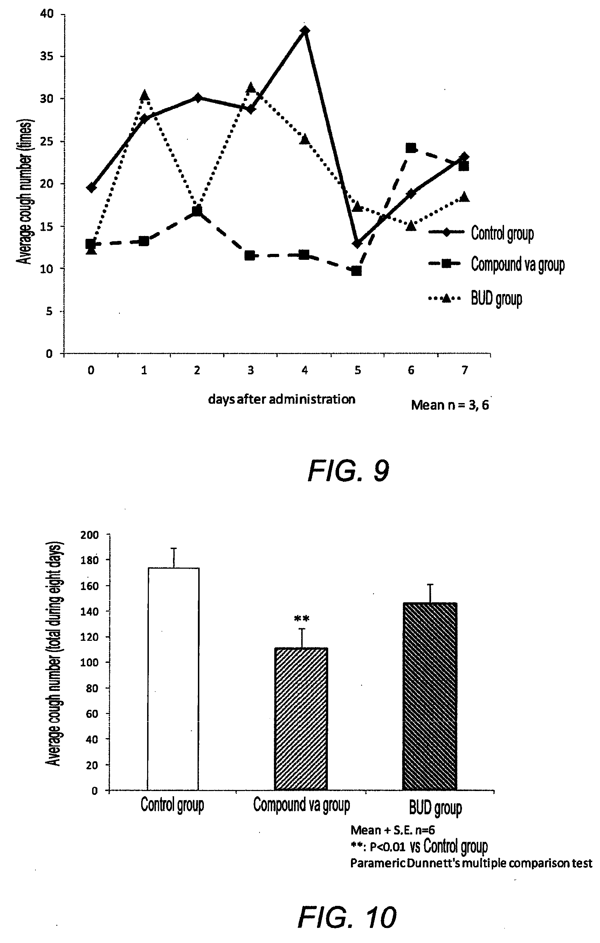

From the first day to Day 7 after inducing initial rhinitis reaction, number of coughs during 10 minutes after the administration of ovalbumin-containing physiological saline was measured. The results are shown in Fig. 9 and Fig. 10.

Meanwhile, on Day 4 and Day 5 after the administration of a test substance, the measurement was performed with example number of 3 for all groups administered with a test substance, and the average cough number on Day 4 and Day 5 after the administration as shown in Fig. 9 was obtained as an average value of those three examples. Furthermore, the average cough number from total 8 days as shown in Fig. 10 was calculated by having the value from a mouse not determined on Day 4 and Day 5 after administration (i.e., not determined with three examples for each administration group) set at zero.

Compared to the group administered with phosphate buffered saline, the group administered with budesonide (BUD group) exhibited inhibited average cough number on the administration day. However, no influence was observed with regard to the change in average cough number from Day 1 to Day 7 after the administration and the total cough number during 8 days. On the other hand, the group administered with the compound va exhibited an inhibition in terms of change in the average cough number and the total cough number during 8 days (Fig. 9 and Fig. 10). However, as the symptom of the rhinitis model was weakened on and after Day 5 after the administration, there was no clear difference between the control group and the group administered with the compound va. Based on above results, a sustaining pharmaceutical effect was confirmed.

Namely, the polysaccharide derivative of the present invention was proven to exhibit a sustaining pharmaceutical effect.

Disclosures of Japanese Patent Application No. 2013-144364 (filing date: July 10,2013) and Japanese Patent Application No. 2013-144365 (filing date: July 10, 2013) are incorporated herein by reference in their entirety.

Regarding all the literatures, patent applications, and technical standards described in the present specification, each of the literatures, patent applications, and technical standards is incorporated herein by reference to the same extent as it is specifically and separately described.

**Claims**

1. A pharmaceutical composition for respiratory administration comprising a polysaccharide derivative having a group derived from a polysaccharide and a group derived from a physiologically active substance that is covalently bonded to the group derived from a polysaccharide.

2. The pharmaceutical composition according to claim 1, wherein the group derived from a polysaccharide and the group derived from a physiologically active substance are covalently bonded with a spacer therebetween.

3. The pharmaceutical composition according to claim 2, wherein the group derived from a physiologically active substance and the spacer are covalently bonded through an ester bond.

4. The pharmaceutical composition according to claim 2 or 3, wherein the group derived from a polysaccharide and the spacer are covalently bonded through an amide bond.

5. The pharmaceutical composition according to any one of claims 2 to 4, wherein the spacer is a divalent or higher valent group that is derived from at least one selected from the group consisting of an amino acid, an amino alcohol, dicarboxylic acid, and a derivative thereof.

6. The pharmaceutical composition according to claim 5, wherein the spacer is a divalent group derived from ω-aminofatty acid which may have a substituent group.

7. The pharmaceutical composition according to any one of claims 1 to 6, wherein the polysaccharide is at least one selected from the group consisting of glycosaminoglycan and homoglycan.

8. The pharmaceutical composition according to any one of claims 1 to 7, wherein the polysaccharide is at least one selected from the group consisting of hyaluronic acid, chondroitin sulfate, chitosan, and carboxymethyl cellulose.

9. The pharmaceutical composition according to any one of claims 1 to 8, wherein dissociation rate of the physiologically active substance from the polysaccharide derivative is 0.1 to 30%/day in phosphate buffered saline with pH of 7.5 at 36°C.

10. The pharmaceutical composition according to any one of claims 1 to 9, wherein the physiologically active substance is at least one selected from the group consisting of a steroid, a bronchodilator, an anti-allergic drug, and an anti-choline drug.

11. The pharmaceutical composition according to any one of claims 1 to 10, which is used for treatment of a respiratory disease.

*FIG. 1*

*FIG. 2*

FIG. 3

Comparison of number of eosinophils in alveolar lavage fluid (%)

FIG. 4

FIG. 5

Data are expressed as the mean values + SE of six animals.

FIG. 6

FIG. 7

mean + S.E. ; n = 13-14
***; P<0.001 vs. Control group, Student's t-test
###; P<0.001 vs. AHR group, Parametric Dunnett's multiple comparison test

FIG. 8

FIG. 9

FIG. 10

## INTERNATIONAL SEARCH REPORT

| International application No. |
| --- |
| PCT/JP2014/068513 |

A.  CLASSIFICATION OF SUBJECT MATTER
*A61K47/48*(2006.01)i, *A61K31/573*(2006.01)i, *A61K47/36*(2006.01)i, *A61K47/38*(2006.01)i, *A61P11/00*(2006.01)i, *A61P11/08*(2006.01)i, *A61P25/02*(2006.01)i, *A61P37/08*(2006.01)i, *A61P43/00*(2006.01)i

According to International Patent Classification (IPC) or to both national classification and IPC

B.  FIELDS SEARCHED

Minimum documentation searched (classification system followed by classification symbols)
A61K47/48, A61K31/573, A61K47/36, A61K47/38, A61P11/00, A61P11/08, A61P25/02, A61P37/08, A61P43/00

Documentation searched other than minimum documentation to the extent that such documents are included in the fields searched
Jitsuyo Shinan Koho        1922-1996    Jitsuyo Shinan Toroku Koho    1996-2014
Kokai Jitsuyo Shinan Koho   1971-2014    Toroku Jitsuyo Shinan Koho    1994-2014

Electronic data base consulted during the international search (name of data base and, where practicable, search terms used)
CAplus(STN)

C.  DOCUMENTS CONSIDERED TO BE RELEVANT

| Category* | Citation of document, with indication, where appropriate, of the relevant passages | Relevant to claim No. |
| --- | --- | --- |
| X<br>Y | Slutter Bram et al., Antigen-Adjuvant Nanoconjugates for Nasal Vaccination: An Improvement over the Use of Nanoparticles?, Molecular Pharmaceutics, 2010, Vol.7, No.6, pp.2207-2215 | 1,2,4,7-9<br>1-11 |
| X<br>Y | Xie Yumei et al., Pulmonary delivery of cisplatin-hyaluronan conjugates via endotracheal instillation for the treatment of lung cancer, International Journal of Pharmaceutics, 2010, Vol.392, No.1-2, pp.156-163 | 1,7-9<br>1-11 |

|☒| Further documents are listed in the continuation of Box C. | |☐| See patent family annex. |

\*      Special categories of cited documents:
"A"    document defining the general state of the art which is not considered    to be of particular relevance
"E"    earlier application or patent but published on or after the international filing date
"L"    document which may throw doubts on priority claim(s) or which is cited to establish the publication date of another citation or other special reason (as specified)
"O"    document referring to an oral disclosure, use, exhibition or other means
"P"    document published prior to the international filing date but later than the priority date claimed

"T"    later document published after the international filing date or priority date and not in conflict with the application but cited to understand the principle or theory underlying the invention
"X"    document of particular relevance; the claimed invention cannot be considered novel or cannot be considered to involve an inventive step when the document is taken alone
"Y"    document of particular relevance; the claimed invention cannot be considered to involve an inventive step when the document is combined with one or more other such documents, such combination being obvious to a person skilled in the art
"&"    document member of the same patent family

| Date of the actual completion of the international search<br>     01 October, 2014 (01.10.14) | Date of mailing of the international search report<br>     14 October, 2014 (14.10.14) |
| --- | --- |
| Name and mailing address of the ISA/<br>     Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2014/068513

C (Continuation). DOCUMENTS CONSIDERED TO BE RELEVANT

| Category* | Citation of document, with indication, where appropriate, of the relevant passages | Relevant to claim No. |
|---|---|---|
| X | JP 2000-501082 A (Hamilton Civic Hospital Research Development Inc.), 02 February 2000 (02.02.2000), entire text; particularly, examples & JP 4166275 B2 & US 6562781 B1 & US 2003/0113314 A1 & US 6491965 B1 & US 2003/0100487 A1 & US 2003/0124705 A1 & EP 863768 A & WO 1997/019701 A2 & DE 69636813 D & DE 69636813 T & AU 7688396 A & CA 2237159 A & AT 350066 T | 1,7,9,11 |
| Y | | 1-11 |
| X | JP 2009-503090 A (Morria Biopharmaceuticals, Yissum Research Development Company of the Hebrew University of Jerusalem), 29 January 2009 (29.01.2009), entire text; particularly, examples & JP 2013-67670 A & JP 5339905 B2 & US 2007/0185052 A1 & US 2014/0100190 A1 & EP 1922076 A & WO 2007/019131 A2 & CA 2617484 A & KR 10-2008-0065269 A & CN 101282733 A & EA 200800489 A & AU 2006278657 A & MX 2008001639 A | 1-9,11 |
| Y | | 1-11 |
| Y | WO 01/05434 A2 (AMGEN INC.), 25 January 2001 (25.01.2001), entire text; particularly, page 9, lines 21 to 27 & AU 6357900 A | 1-11 |
| Y | JP 2009-508852 A (Kwangju Institute of Science and Technology), 05 March 2009 (05.03.2009), entire text; particularly, paragraphs [0031] to [0041] & JP 2012-51946 A & US 2007/0292387 A1 & EP 1973952 A & WO 2007/083984 A1 & KR 10-0791414 B1 & KR 10-2007-0077428 A & CN 101405301 A | 1-11 |
| Y | JP 2003-507344 A (PARK, Myung-Ok, LEE, Kang, Choon), 25 February 2003 (25.02.2003), entire text; particularly, claim 5 & US 6506730 B1 & EP 1204427 A & WO 2001/012230 A1 & KR 10-2001-0018158 A & AU 6478400 A | 1-11 |

Form PCT/ISA/210 (continuation of second sheet) (July 2009)

**INTERNATIONAL SEARCH REPORT**

International application No.

PCT/JP2014/068513

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 2004/000863 A1  (Nippon Suisan Kaisha, Ltd.),<br>31 December 2003 (31.12.2003),<br>entire text; particularly, claim 11; page 10<br>& US 2005/0203061 A1    & EP 1541579 A1<br>& CA 2490626 A          & BR 312442 A<br>& NZ 537686 A           & AU 2003244088 A<br>& MX PA05000028 A       & KR 10-2005-0027095 A<br>& CN 1671724 A          & ZA 200500526 A | 1-11 |

Form PCT/ISA/210 (continuation of second sheet) (July 2009)

**REFERENCES CITED IN THE DESCRIPTION**

**Patent documents cited in the description**

- JP 2008156327 A
- JP 2013144364 A

- JP 2013144365 A

**Non-patent literature cited in the description**

- **MATSUYAMA MOTOTAKA ; SHUMOKU TOMOYUKI ; ONISHI HIRAKU.** Preparation of Chondroitin sulfate-Prednisolone Conjugate and In Vitro Characteristics. *27th Lecture Synopsis of The Academy of Pharmaceutical Science and Technology,* 24 May 2012, 24-12